# EUROPEAN PATENT APPLICATION

(11) **EP 3 796 249 A1**
(43) Date of publication of application: **24.03.2021**
(21) Application number: 19803974.5
(22) Date of filing: 26.04.2019
(51) Int. Cl.: G06Q 40/08, G16H 50/30

(54) **SPECIMEN EXAMINATION MANAGEMENT DEVICE, SPECIMEN EXAMINATION MANAGEMENT SYSTEM, SPECIMEN EXAMINATION MANAGEMENT METHOD, AND PROGRAM**

(30) Priority: 15.05.2018 JP 2018093816
(71) Applicant: FUJIFILM CORPORATION, Minato-ku Tokyo 106-8620 (JP); Leisure, Inc., Tokyo 103-0013 (JP)
(72) Inventor: KANEKO, Yasuhisa, Ashigarakami-gun, Kanagawa 258-8538 (JP); SUZUKI, Masakazu, Tokyo 103-0013 (JP)
(74) Representative: Jeffrey, Philip Michael
(86) International application number: PCT/JP2019/018017
(87) International publication number: WO 2019/220939

(57) **Abstract**

Provided are a specimen examination management device, a specimen examination management system, a specimen examination management method, and a program that allow an insurance subscriber to receive a proposition of an insurance product without an examination, and furthermore that make it possible to handle a proposition of an insurance premium corresponding to individual health consciousness. There are included: an examination result acquisition unit (30A) configured to acquire a result of a specimen examination using a specimen examination kit for a specimen examination subject; a health degree computation unit (34) configured to derive a health degree of the specimen examination subject based on the result of the specimen examination, the health degree indicating a health state related to insurance subscription; an insurance information acquisition unit (32B) configured to acquire insurance information including an insurance premium based on the health degree of the specimen examination subject, from an insurance premium calculation device configured to calculate the insurance premium based on the health degree; an insurance proposition creation unit (34) configured to create an insurance proposition including the insurance information; and an insurance proposition output unit (32B) configured to output the insurance proposition.

## Description

### {Technical Field}

The present invention relates to a specimen examination management device, a specimen examination management system, a specimen examination management method, and a program, and particularly relates to management of a specimen examination.

### {Background Art}

With technological evolution, made-to-order services to respond to individual needs have increased. As an example of the made-to-order services, there is made-to-order medicine. For example, a medication administration or the like that agrees with the constitution, physical condition and others of each person is performed based on an SNPs analysis in a gene examination. Here, SNPs is an abbreviation of single nucleotide polymorphisms.

Also as for insurance, the application of IT is advancing, and a detailed insurance product line depending on individuals is prepared. For example, there is an insurance for which an insurance premium is set depending on blood examination item values such as a BMI value, a blood pressure value and a GOT value, whether the individual is a smoker, and the like.

In the insurance for which the insurance premium is set depending on individual conditions, a subscriber performs an examination in a hospital or the like, and the subscriber performs self-report of an examination result. Here, IT is an abbreviation of Information Technology. BMI is an abbreviation of Body Mass Index. GOT represents glutamic oxaloacetic transaminase.

PTL 1 describes an insurance system in which an all-cancer risk evaluation is performed based on a result of an all-cancer examination with reference to an all-cancer risk evaluation model and the insurance premium is calculated based on an all-cancer risk evaluation result.

### {Citation List}

### {Patent Literature}

{PTL 1} Japanese Patent Application Laid-Open No. 2017-134474

### {Summary of Invention}

### {Technical Problem}

Recently, because of increase in health consciousness, an interest in fitness, diet and the like has increased, and an increasing number of people actively perform health maintenance. However, a proposition of an insurance product corresponding to an individual health consciousness has not been handled sufficiently. Further, the insurance subscriber cannot receive a proposition of an insurance product without the examination in a hospital or the like.

The invention described in PTL 1 does not handle a proposition of an insurance premium corresponding to an individual health consciousness, although the all-cancer risk evaluation is performed from the result of the all-cancer examination and the insurance premium is calculated based on the evaluation result of the all-cancer risk.

The present invention has been made in view of such a circumstance, and has an object to provide a specimen examination management device, a specimen examination management system, a specimen examination management method, and a program that allow the insurance subscriber to receive the proposition of the insurance product without the examination, and furthermore that make it possible to handle the proposition of the insurance premium corresponding to the individual health consciousness.

### {Solution to Problem}

For achieving the above object, the following invention aspects are provided.

A specimen examination management device according to a first aspect is a specimen examination management device including: an examination result acquisition unit configured to acquire a result of a specimen examination using a specimen examination kit for each specimen examination subject; a health degree computation unit configured to derive a health degree of the specimen examination subject based on the result of the specimen examination of the specimen examination subject that is acquired from the examination result acquisition unit, the health degree indicating a health state related to insurance subscription; an insurance information acquisition unit configured to acquire insurance information including an insurance premium based on the health degree of the specimen examination subject, from an insurance premium calculation device configured to calculate the insurance premium based on the health degree; an insurance proposition creation unit configured to create an insurance proposition including the insurance information that is acquired using the insurance information acquisition unit; and an insurance proposition output unit configured to output the insurance proposition that is created using the insurance proposition creation unit.

According to the first aspect, the insurance information including the insurance premium based on the health degree of the specimen examination subject that indicates the health state related to the insurance subscription is acquired, the insurance proposition is created based on the insurance information, and the proposition is performed. Thereby, it is possible to handle the proposition of the insurance premium corresponding to the individual health consciousness.

The specimen examination subject can include at least one of a person that applies for the specimen examination oneself and undergoes the specimen examination and a person that undergoes the specimen examination while another person such as a family member applies.

As the specimen, a specimen that can be taken using the specimen examination kit, as exemplified by blood, stool, urine, hair, and skin, is applied. As the specimen, a human specimen may be applied, or an animal other than human may be applied.

The insurance means an insurance contract in which an insurance subscriber pays the insurance premium and receives a security such as security money payment from an insurance company when a prescribed condition is satisfied. As examples of the insurance, there are life insurance, medical insurance, accident insurance and the like.

The specimen examination management device according to the first aspect includes one or more processors and one or more memories. The one or more processors acquire the result of the specimen examination using the specimen examination kit for each specimen examination subject, derive the health degree of the specimen examination subject that indicates the health state related to the insurance subscription, based on the result of the specimen examination of the specimen examination subject that is acquired from the examination result acquisition unit, acquire the insurance information including the insurance premium based on the health degree of the specimen examination subject, from the insurance premium calculation device that calculates the insurance premium based on the health degree, and create the insurance proposition including the insurance information that is acquired using the insurance information acquisition unit. The memory can function as a specimen examination management device that stores data in each process.

In a second aspect, in the specimen examination management device according to the first aspect, the health degree computation unit may be configured to compute, as the health degree, an age difference resulting from subtracting a real age of the specimen examination subject from a health state age resulting from converting the health state of the specimen examination subject into an age.

According to the second aspect, it is possible to calculate the insurance premium based on the age difference resulting from subtracting the real age from the health state age.

In a third aspect, in the specimen examination management device according to the second aspect, the examination result acquisition unit may be configured to acquire a result of a blood examination that adopts, as an examination item, at least one of glycohemoglobin, estimated glomerular filtration rate, albumin, aspartate aminotransferase, low-density lipoprotein cholesterol, alkaline phosphatase and total cholesterol, and the health degree computation unit may be configured to derive the health state age, from at least the examination item.

According to the third aspect, it is possible to derive the health degree using the health state age that is derived based on the result of the blood examination.

As an example of a blood examination kit that is applied to the blood examination, there is a blood examination kit including: a blood taking member by which blood is taken; a container in which a dilution buffer solution for dispersing the blood taken using the blood taking member is enclosed; a separation member that separates the blood dispersed in the dilution buffer solution into blood plasma and blood cells; and a sealing cap that is attached to the container and that seals the container.

In a fourth aspect, in the specimen examination management device according to any one aspect of the first aspect to the third aspect, the examination result acquisition unit may be configured to acquire the result of the specimen examination of the specimen examination subject, from an examination result storage device configured to store the result of the specimen examination using the specimen examination kit for each specimen examination subject.

According to the fourth aspect, it is possible to acquire the result of the specimen examination of the specimen examination subject, from the examination result storage device.

In the fifth aspect, the specimen examination management device according to the fourth aspect may be configured to include the examination result storage device.

According to the fifth aspect, it is possible to acquire the result of the specimen examination of the specimen examination subject, from the examination result storage device equipped in the specimen examination management device.

In a sixth aspect, the specimen examination management device according to the fifth aspect may be configured to include a personal authentication information acquisition unit configured to acquire personal authentication information about the specimen examination subject, in which the examination result storage device is configured to store the personal authentication information about the specimen examination subject that is acquired using the personal authentication information acquisition unit, in association with the result of the specimen examination.

According to the sixth aspect, the result of the specimen examination associated with the personal authentication information can be used as contract assessment information for determining whether the insurance subscription is permitted, at the time of the insurance contract such as the insurance subscription and insurance renewal.

The personal authentication information acquisition unit can acquire the personal authentication information that is sent from the specimen examination subject terminal device. The specimen examination subject terminal device can send the personal authentication information about the specimen examination subject based on pickup data that is obtained by picking up the specimen examination subject or an identification certificate of the specimen examination subject using a pickup device.

In a seventh aspect, the specimen examination management device according to the fifth aspect or the sixth aspect may be configured to include a medical measurement value acquisition unit configured to acquire a medical measurement value of the specimen examination subject that is measured using a medical measurement apparatus, in which the examination result storage device is configured to store the medical measurement value of the specimen examination subject that is acquired using the medical measurement value acquisition unit, in association with the result of the specimen examination.

According to the seventh aspect, it is possible to use information about the medical measurement value at the time of the insurance contract such as the insurance renewal.

As an example of the medical measurement apparatus, there is a simplified medical measurement apparatus that is operated by a measurer oneself and that measures blood pressure or the like.

In an eighth aspect, the specimen examination management device according to any one aspect of the first aspect to the seventh aspect may be configured to include a health degree information output unit configured to output health degree information including the health degree, to the insurance premium calculation device.

According to the eighth aspect, it is possible to output the health degree information to the insurance premium calculation device.

In a ninth aspect, in the specimen examination management device according to any one aspect of the first aspect to the eighth aspect, the insurance proposition output unit may be configured to output the insurance proposition to a subject terminal device that is used by the specimen examination subject.

According to the ninth aspect, the specimen examination subject can recognize the insurance proposition using the subject terminal device.

In a tenth aspect, the specimen examination management device according to the ninth aspect may be configured to include an examination result output unit configured to output the examination result that is acquired using the examination result acquisition unit, to the subject terminal device.

According to the tenth aspect, the specimen examination subject can recognize the examination result using the subject terminal device.

In an eleventh aspect, the specimen examination management device according to the ninth aspect or the tenth aspect may be configured to include: an intention confirmation information output unit configured to output intention confirmation information to the subject terminal device during an insurance contract continuation procedure allowing period, the intention confirmation information being information for confirming continuation intention for an insurance contract, the insurance contract continuation procedure allowing period being a period in which a continuation procedure of the insurance contract is allowed; an intention information acquisition unit configured to acquire intention information indicating the continuation intention for the insurance contract of the specimen examination subject, the intention information being output from the subject terminal device; and a delivery information output unit configured to output delivery information to a providing organization of the specimen examination kit, when the intention information acquisition unit acquires the intention information, the delivery information including a delivery address of the specimen examination kit for the specimen examination subject that outputs the intention information.

According to the eleventh aspect, when the intention information indicating the intention of the insurance continuation is acquired from the subject terminal, it is possible to deliver the specimen examination kit to the specimen examination subject. Thereby, the specimen examination is performed at the time of the insurance continuation, and the health degree based on the specimen examination result at the time of the insurance continuation is derived, allowing the calculation of the insurance premium based on the health degree at the time of the insurance continuation.

In a twelfth aspect, in the specimen examination management device according to the eleventh aspect, the delivery information output unit may be configured to output the delivery information to the providing organization of the specimen examination kit, when an insurance premium of an insurance after the insurance contract is continued is equal to or more than an insurance premium of an insurance under the contract, the delivery information including the delivery address of the specimen examination kit for the specimen examination subject that hopes for continuation of the insurance contract.

According to the twelfth aspect, it is possible to deliver the specimen examination kit to the specimen examination subject, when the insurance premium of the insurance after the insurance contract is continued is equal to or more than the insurance premium of the insurance under the contract. Thereby, the specimen examination is performed in the case of being equal to or more than the insurance premium of the insurance under contract, and the health degree based on the specimen examination result is derived, allowing the calculation of the insurance premium based on the health degree.

In a thirteenth aspect, in the specimen examination management device according to the eleventh aspect or the twelfth aspect, the insurance information acquisition unit may be configured to acquire information about an insurance premium of an insurance having a different contract condition including shortening of an insurance period, when an insurance premium of an insurance after the insurance contract is continued is equal to or more than an insurance premium of an insurance under the contract.

According to the thirteenth aspect, it is possible to propose the insurance premium of the insurance having the different contract condition including the shortening of the insurance period, to the specimen examination subject, when the insurance premium of the insurance after the insurance contract is continued is equal to or more than the insurance premium of the insurance under the contract.

A specimen examination management system according to a fourteenth aspect is a specimen examination management system including a server device configured to be capable of being connected with a network, the server device including: an examination result acquisition unit configured to acquire a result of a specimen examination using a specimen examination kit for each specimen examination subject; a health degree computation unit configured to derive a health degree of the specimen examination subject based on the result of the specimen examination of the specimen examination subject that is acquired from the examination result acquisition unit, the health degree indicating a health state related to insurance subscription; an insurance information acquisition unit configured to acquire information about an insurance premium based on the health degree of the specimen examination subject, from an insurance premium calculation device configured to calculate the insurance premium based on the health degree; an insurance proposition creation unit configured to create an insurance proposition including the information about the insurance premium that is acquired using the insurance information acquisition unit; and an insurance proposition output unit configured to output the insurance proposition that is created using the insurance proposition creation unit.

According to the fourteenth aspect, it is possible to obtain the same effect as the first aspect.

In the fourteenth aspect, the same matters as the matters specified in the second aspect to the thirteenth aspect can be combined when appropriate. In that case, a constituent element that has a process and function specified in the specimen examination management device can be regarded as a constituent element of the specimen examination management system that has the corresponding process and function.

In a fifteenth aspect, the specimen examination management system according to the fourteenth aspect may be configured to include an examination result storage device configured to be capable of being connected with the network, in which: the examination result storage device is configured to store the result of the specimen examination using the specimen examination kit for each specimen examination subject; and the examination result acquisition unit is configured to acquire the result of the specimen examination of the specimen examination subject, from the examination result storage device.

According to the fifteenth aspect, it is possible to acquire the result of the specimen examination of the specimen examination subject from the examination result storage device.

In a sixteenth aspect, the specimen examination management system according to the fourteenth aspect or the fifteenth aspect may be configured to include a subject terminal device configured to be capable of being connected with the network, the subject terminal device being used by the specimen examination subject, in which: the subject terminal device includes a display unit configured to display information that is output from the server device; and the display unit is configured to display the insurance proposition that is output from the server device.

According to a sixteenth aspect, it is possible to recognize the insurance proposition that is output from the server device, using the display unit of the subject terminal device.

In a seventeenth aspect, in the specimen examination management system according to the sixteenth aspect, the display unit may be configured to display intention confirmation information during an insurance contract continuation procedure allowing period, the intention confirmation information being information for confirming continuation intention for an insurance contract that is output from the server device, the insurance contract continuation procedure allowing period being a period in which a continuation procedure of the insurance contract is allowed.

According to the seventeenth aspect, the specimen examination subject can recognize the intention confirmation information that is output from the server device, using the display unit of the subject terminal device. Thereby, the specimen examination subject can output information indicating whether to continue the insurance, to the server device, using the subject terminal device.

In an eighteenth aspect, in the specimen examination management system according to the sixteenth aspect or the seventeenth aspect, the display unit may be configured to display selection information that is output from the server device, when an insurance premium of an insurance after the insurance contract is continued is equal to or more than an insurance premium of an insurance under the contract, the selection information being information for selecting whether to perform a re-examination.

According to the eighteenth aspect, the specimen examination subject can recognize the selection information that is output from the server device, using the display unit of the subject terminal device. Thereby, the specimen examination subject can output information indicating whether to perform the re-examination, to the server device, using the subject terminal device.

In a nineteenth aspect, in the specimen examination management system according to any one aspect of the sixteenth aspect to the eighteenth aspect, the display unit may be configured to display information that is output from the server device, when an insurance premium of an insurance after the insurance contract is continued is equal to or more than an insurance premium of an insurance under the contract, the information being information about an insurance premium of an insurance having a different contract condition including shortening of an insurance period.

According to the nineteenth aspect, the specimen examination subject can recognize the information about the insurance premium of the insurance having the different contract including the shortening of the insurance period, which is output from the server device, using the display unit of the subject terminal device.

A specimen examination management method according to a twentieth aspect includes: an examination result acquisition step of acquiring a result of a specimen examination using a specimen examination kit for each specimen examination subject; a health degree computation step of deriving a health degree of the specimen examination subject based on the result of the specimen examination of the specimen examination subject that is acquired in the examination result acquisition step, the health degree indicating a health state related to insurance subscription; an insurance information acquisition step of acquiring insurance information including an insurance premium based on the health degree of the specimen examination subject, from an insurance premium calculation device configured to calculate the insurance premium based on the health degree; an insurance proposition creation step of creating an insurance proposition including information about the insurance premium that is acquired in the insurance information acquisition step; and an insurance proposition output step of outputting the insurance proposition that is created in the insurance proposition creation step.

According to the twentieth aspect, it is possible to obtain the same effect as the first aspect.

In the twentieth aspect, the same matters as the matters specified in the second aspect to the thirteenth aspect and in the fifteenth aspect to the nineteenth aspect can be combined when appropriate. In that case, a constituent element that has a process and function specified in the specimen examination management device and the specimen examination management system can be regarded as a constituent element of the specimen examination management method that has the corresponding process and function.

A program according to a twenty-first aspect is a program causing a computer to realize: an examination result acquisition function to acquire a result of a specimen examination using a specimen examination kit for each specimen examination subject; a health degree computation function to derive a health degree of the specimen examination subject based on the result of the specimen examination of the specimen examination subject that is acquired using the examination result acquisition function, the health degree indicating a health state related to insurance subscription; an insurance information acquisition function to acquire insurance information including an insurance premium based on the health degree of the specimen examination subject, from an insurance premium calculation device configured to calculate the insurance premium based on the health degree; an insurance proposition creation function to create an insurance proposition including information about the insurance premium that is acquired using the insurance information acquisition function; and an insurance proposition output function to output the insurance proposition that is created using the insurance proposition creation function.

According to the twenty-first aspect, it is possible to obtain the same effect as the first aspect.

In the twenty-first aspect, the same matters as the matters specified in the second aspect to the thirteenth aspect and in the fifteenth aspect to the nineteenth aspect can be combined when appropriate. In that case, a constituent element that has a process and function specified in the specimen examination management device, the specimen examination management system, and the specimen examination management method can be regarded as a constituent element of the program that has the corresponding process and function.

A specimen examination management device according to a twenty-second aspect includes: a health degree computation unit configured to derive a health degree of a specimen examination subject based on at least one of a result of a specimen examination of the specimen examination subject and specimen examination subject information, the health degree indicating a health state related to insurance subscription, the specimen examination subject information being information related to the specimen examination subject; an insurance information acquisition unit configured to acquire insurance information including an insurance premium based on the health degree of the specimen examination subject, from an insurance premium calculation device configured to calculate the insurance premium based on the health degree; an insurance proposition creation unit configured to create an insurance proposition including the insurance information that is acquired using the insurance information acquisition unit; and an insurance proposition output unit configured to output the insurance proposition that is created using the insurance proposition creation unit.

According to the twenty-second aspect, it is possible to obtain the same effect as the first aspect.

In the twenty-second aspect, the same matters as the matters specified in the second aspect to the thirteenth aspect and in the fifteenth aspect to the nineteenth aspect can be combined when appropriate.

In a twenty-third aspect, in the specimen examination management device according to the twenty-second aspect, the specimen examination subject information may be configured to include at least one of body information relevant to a body of the specimen examination subject and taste information relevant to a taste of the specimen examination subj ect.

### {Advantageous Effects of Invention}

According to the present invention, the insurance information including the insurance premium based on the health degree of the specimen examination subject that indicates the health state related to the insurance subscription is acquired, the insurance proposition is created based on the insurance information, and the proposition is performed. Thereby, it is possible to handle the proposition of the insurance premium corresponding to the individual health consciousness.

### {Brief Description of Drawings}

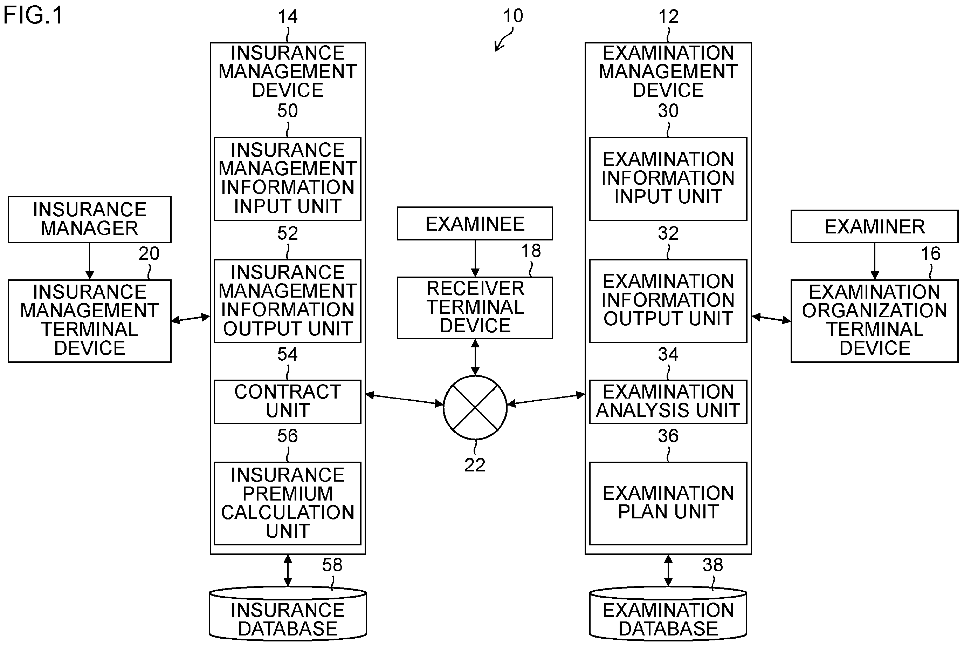

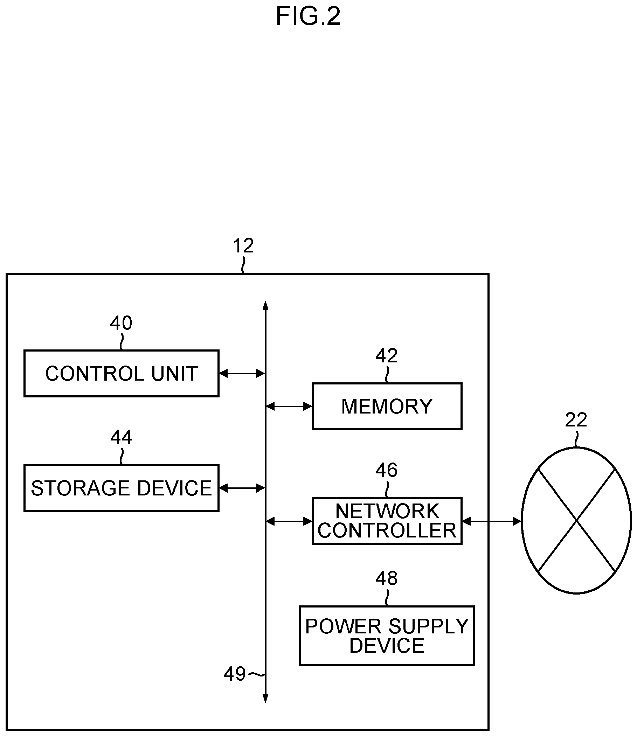

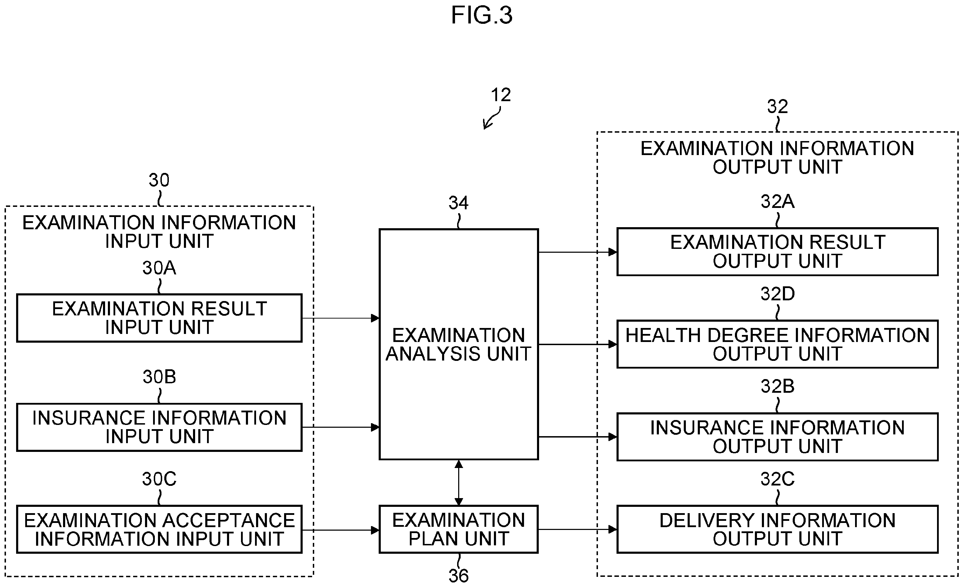

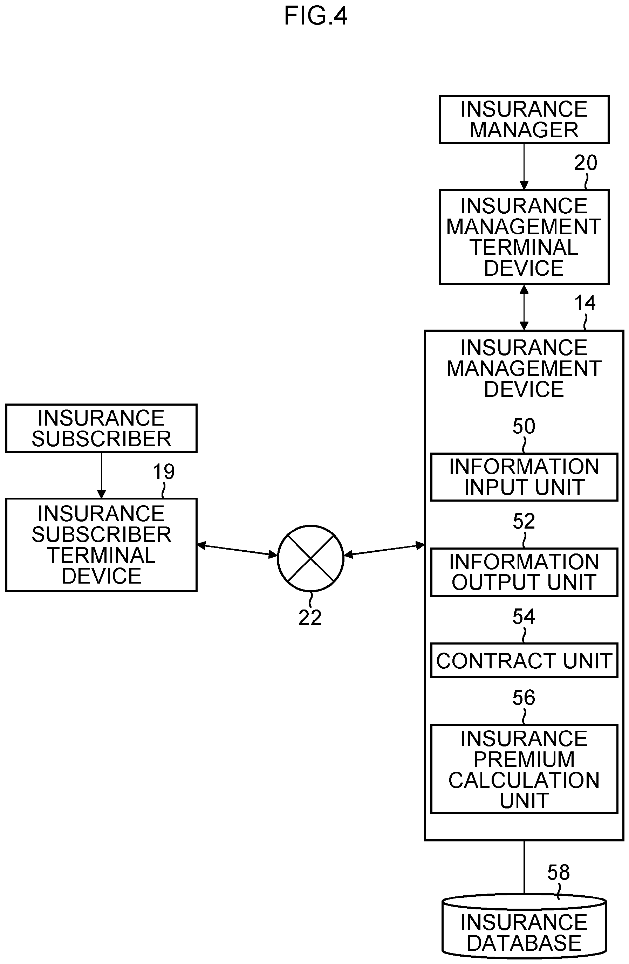

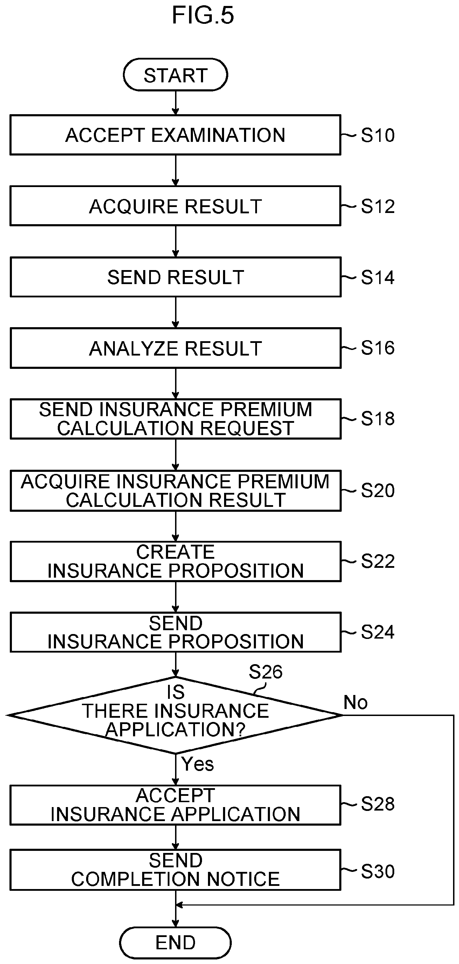

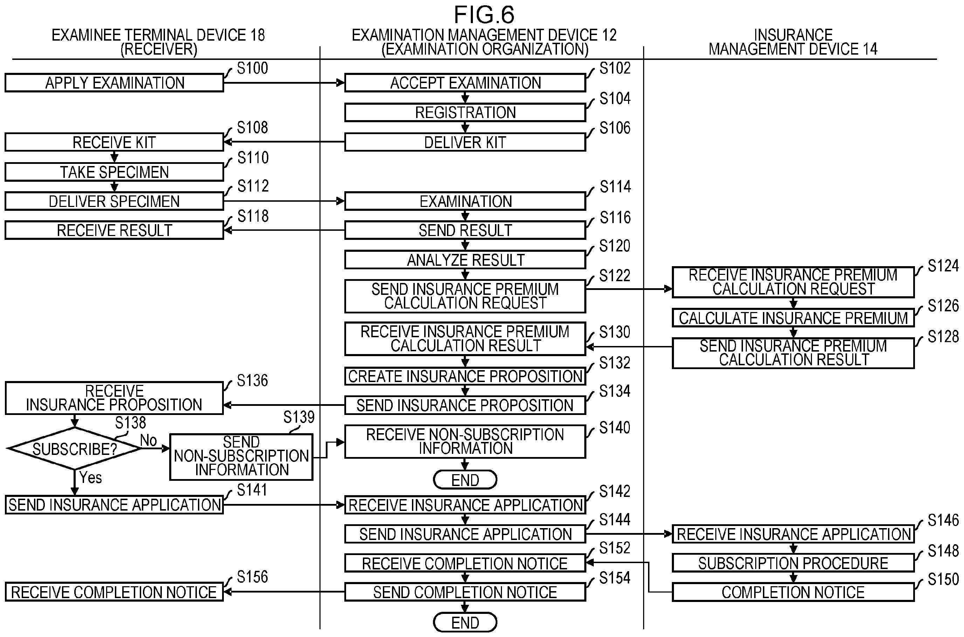

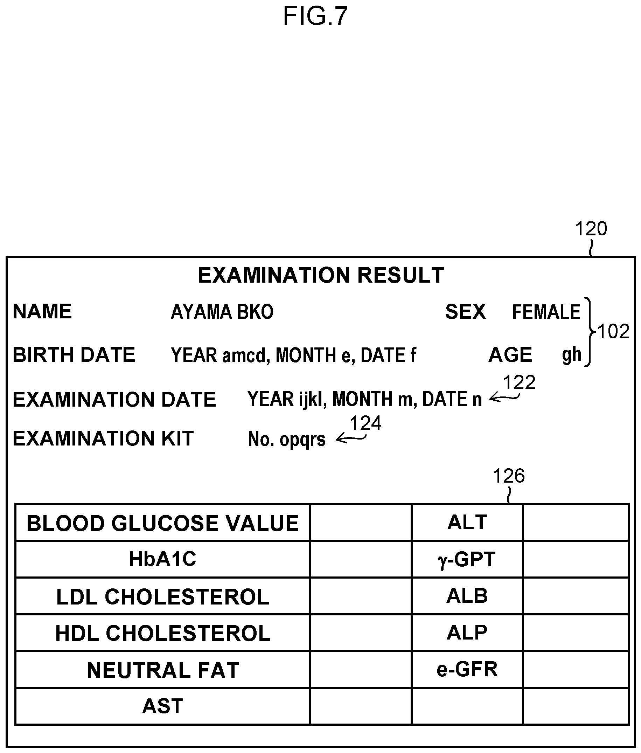

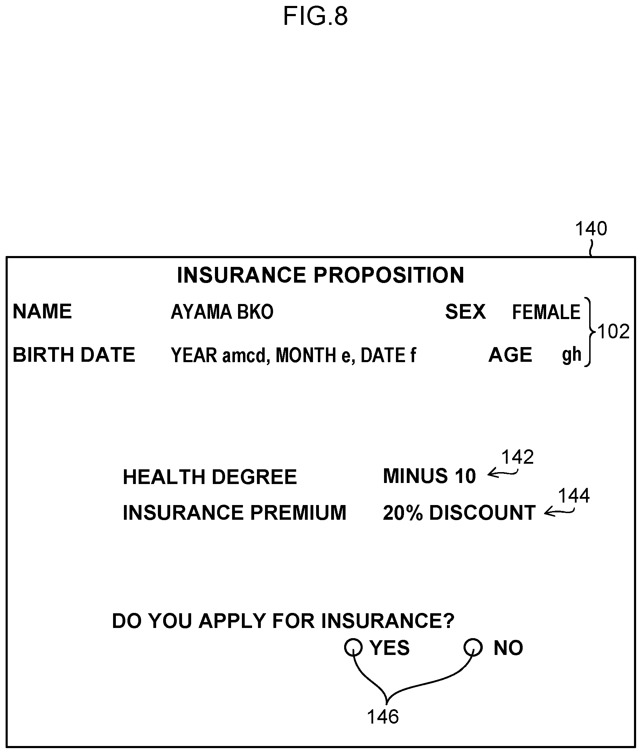

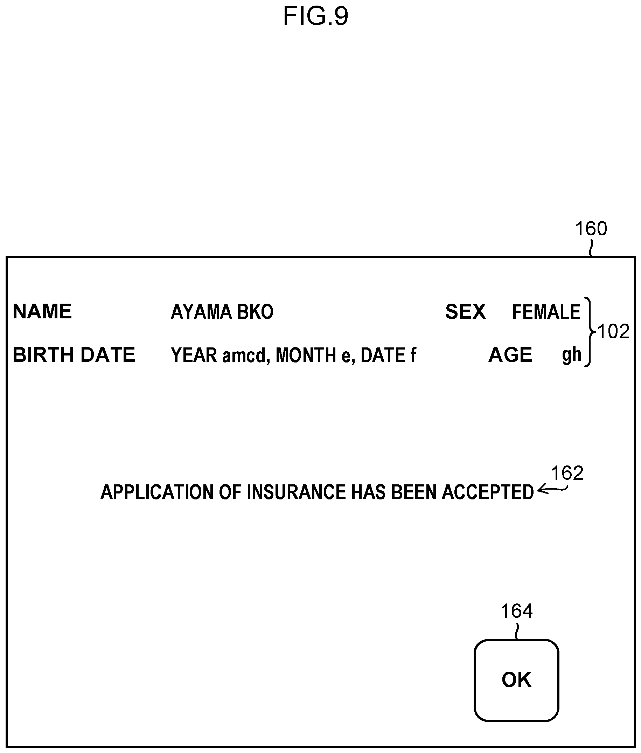

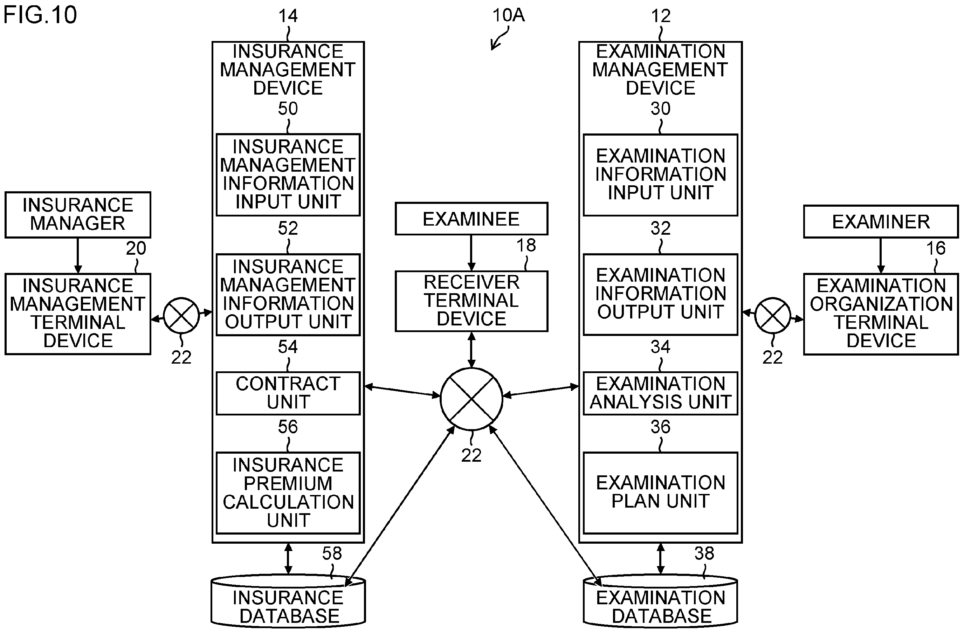

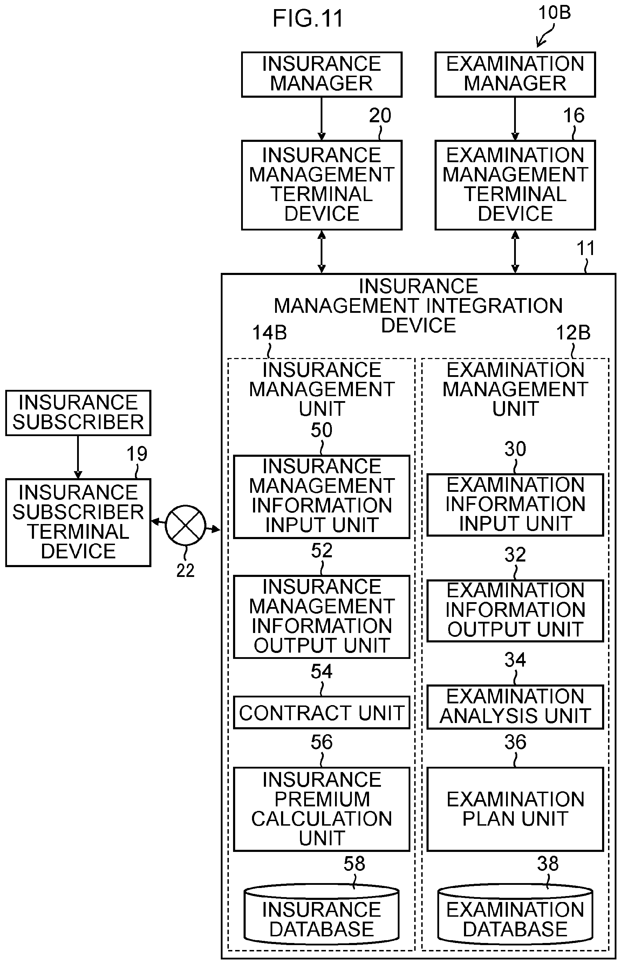

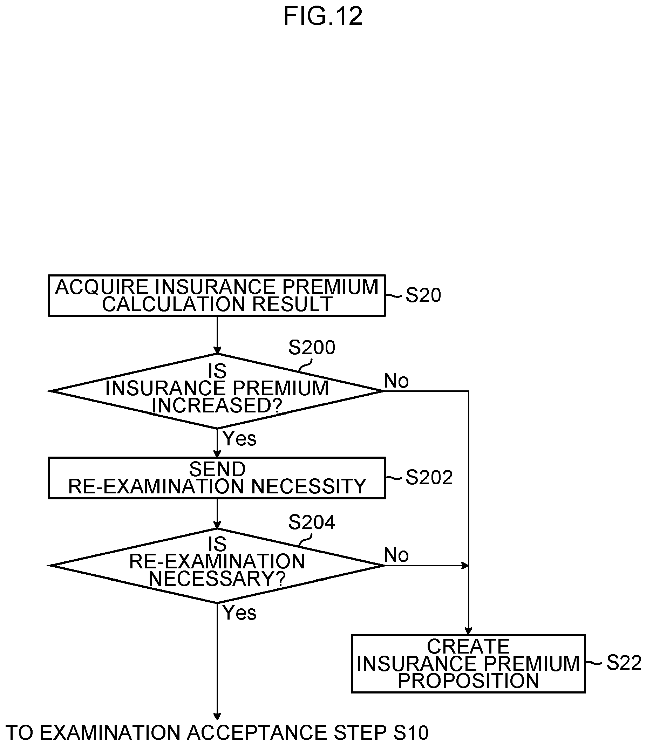

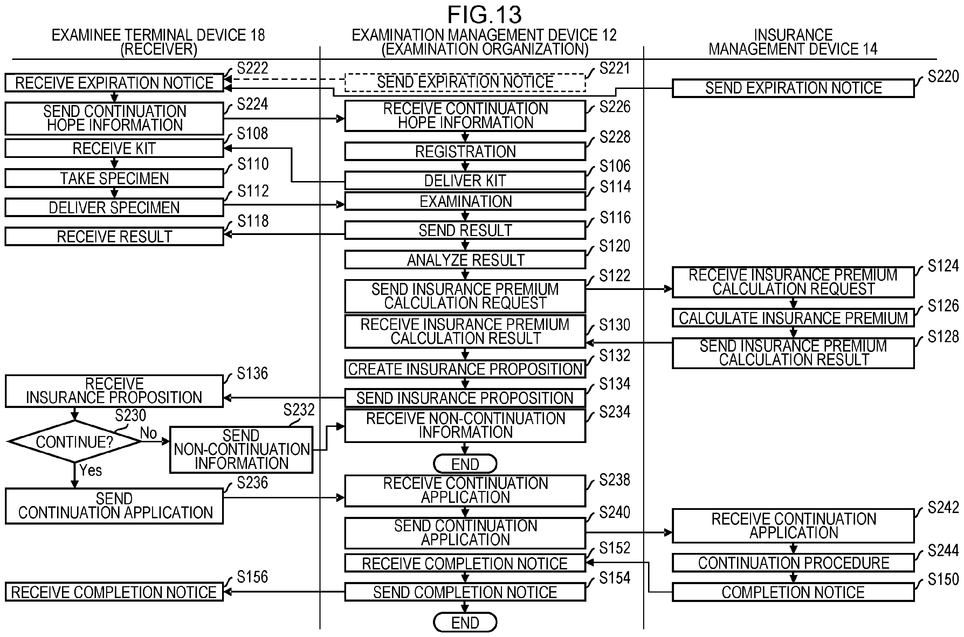

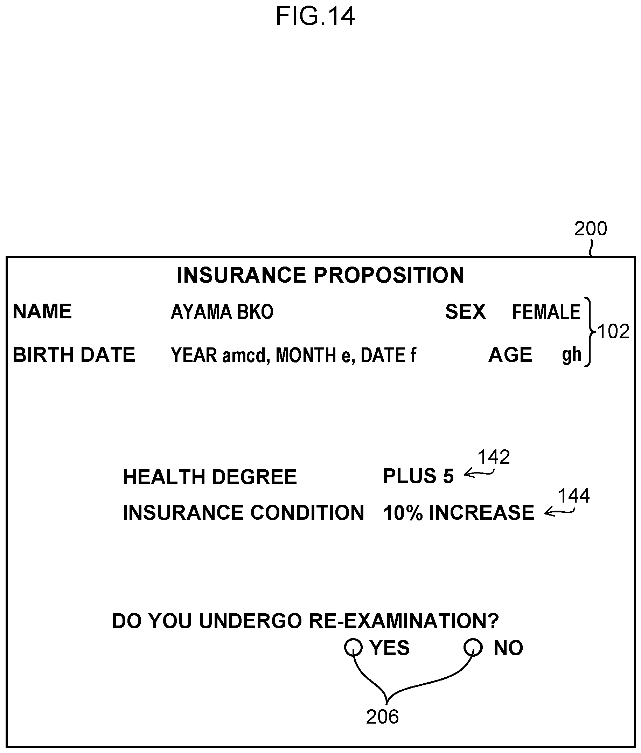

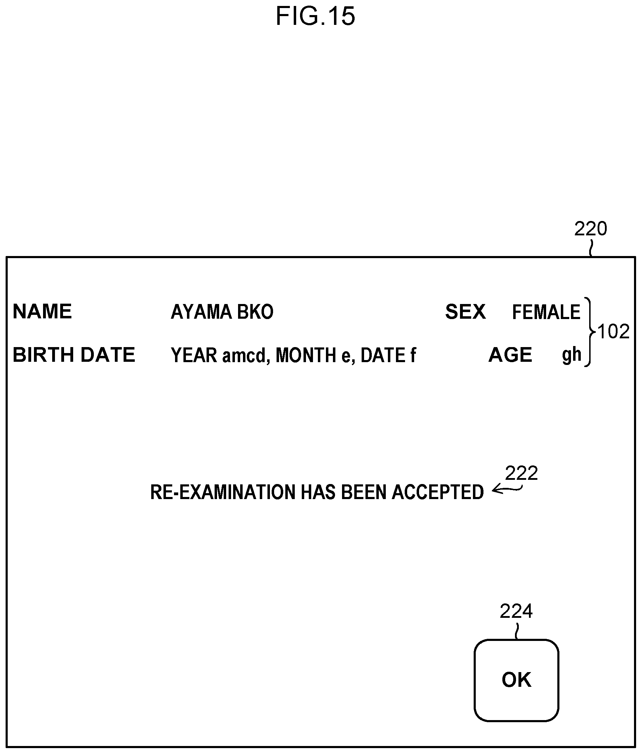

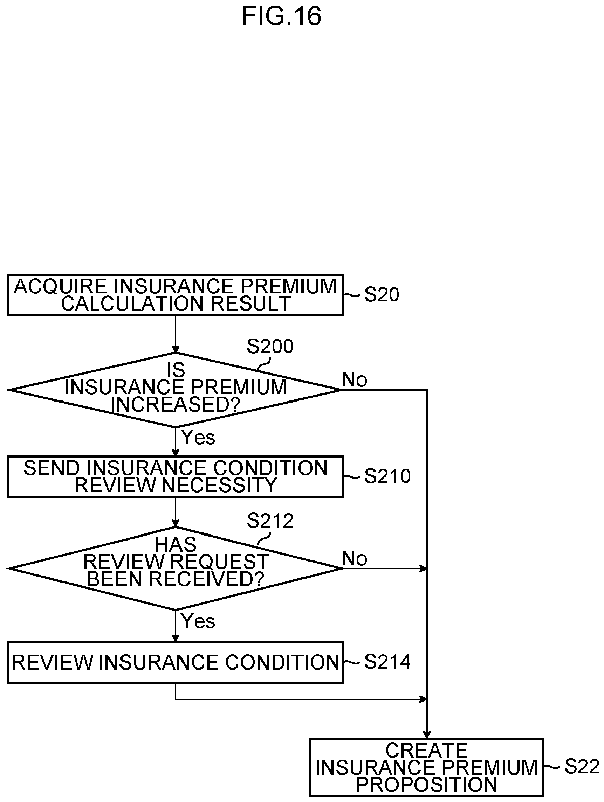

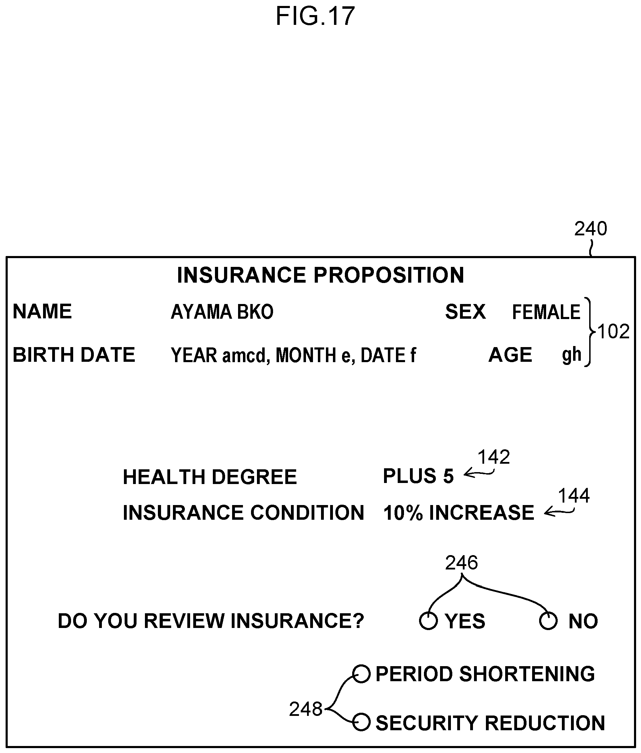

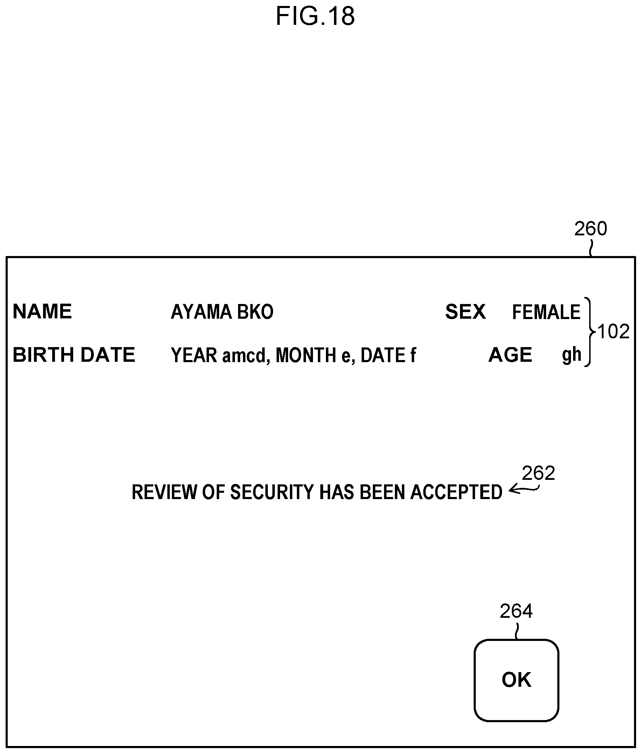

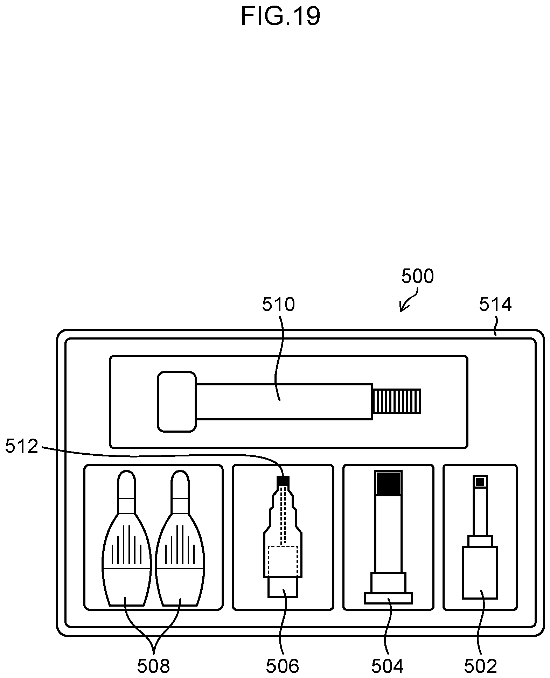

{Figure 1} Figure 1 is a block diagram showing a configuration example of a specimen examination management system according to a first embodiment.
{Figure 2} Figure 2 is a block diagram showing a configuration example of hardware of an examination management device.
{Figure 3} Figure 3 is a functional block diagram of the examination management device.
{Figure 4} Figure 4 is a block diagram showing a connection example of an insurance management device and an insurance subscriber terminal.
{Figure 5} Figure 5 is a flowchart showing a flow of a sequence of a specimen examination management method according to the first embodiment.
{Figure 6} Figure 6 is a flowchart showing a flow of information among constituent elements of the specimen examination management system and a flow of a blood examination kit.
{Figure 7} Figure 7 is an explanatory diagram showing an example of an examination result screen.
{Figure 8} Figure 8 is an explanatory diagram showing an example of an insurance proposition screen.
{Figure 9} Figure 9 is an explanatory diagram showing an example of an application completion screen.
{Figure 10} Figure 10 is a block diagram showing a configuration example of a specimen examination management system to which a network system is applied.
{Figure 11} Figure 11 is a block diagram showing a configuration example of a specimen examination management system according to a modification.
{Figure 12} Figure 12 is a flowchart showing a flow of a sequence of a specimen examination management method according to a second embodiment.
{Figure 13} Figure 13 is a flowchart showing a flow of information among constituent elements of a specimen examination management system and a flow of a blood examination kit in the specimen examination management method according to the second embodiment.
{Figure 14} Figure 14 is an explanatory diagram showing an example of an insurance proposition screen including a re-examination acceptance.
{Figure 15} Figure 15 is an explanatory diagram showing an example of a re-examination acceptance completion screen.
{Figure 16} Figure 16 is a flowchart showing a flow of a specimen examination management method according to an application example of the second embodiment.
{Figure 17} Figure 17 is an explanatory diagram showing an example of an insurance proposition screen including an insurance condition review selection.
{Figure 18} Figure 18 is an explanatory diagram showing an example of an insurance condition review acceptance completion screen.
{Figure 19} Figure 19 is a configuration diagram of a blood examination kit.

### {Description of Embodiments}

Hereinafter, preferable embodiments of the present invention are described in detail with the accompanying drawings. In the present specification, identical constituent elements are designated by identical reference characters, and repetitive descriptions are omitted when appropriate.

### [Specimen Examination Management System According to First Embodiment]

### [Overall Configuration]

Figure 1 is a block diagram showing a configuration example of a specimen examination management system according to a first embodiment. For an examinee that hopes for a specimen examination using a specimen examination kit, a specimen examination management system 10 shown in Figure 1 calculates an insurance premium based on an examination result, and performs an insurance proposition including the insurance premium. For example, when the examination result of the examinee is good, the specimen examination management system 10 proposes how much the insurance premium can be reduced, to the examinee. The insurance premium may include a specimen examination cost.

In the embodiment, life insurance is described as an example. However, the embodiment can be applied to any insurance in which the insurance premium is decided depending on the health state of an insurance subscriber that is known from the specimen examination result, for example, to medical insurance and accident insurance. Herein, the insurance subscriber is synonymous with the examinee that undergoes the specimen examination.

The specimen examination management system 10 shown in Figure 1 includes an examination management device 12, an insurance management device 14, an examination organization terminal device 16, an examinee terminal device 18, and an insurance management terminal device 20. The examination management device 12, the insurance management device 14, and the examinee terminal device 18 are connected through a network 22 in a communicable manner.

### [Examination Management Device]

The examination management device 12 is connected with the examination organization terminal device 16 and an examination database 38 in a communicable manner. The examination management device 12 may include the examination database 38. The connection form of the examination management device 12 with the examination organization terminal device 16 and the examination database 38 may be a connection using wires or a connection using no wire.

The examination management device 12 and the examination database 38 may be disposed at an examination organization, or may be disposed at a different place from the examination organization. Further, the examination management device 12 and the examination database 38 may be disposed at an identical place, or may be disposed at different places. The different places may include separated different areas in an identical building. Here, the examination management device 12 corresponds to an example of the specimen examination management device.

The examination management device 12 includes an examination information input unit 30, an examination information output unit 32, an examination analysis unit 34, and an examination plan unit 36. The examination management device 12 includes an unillustrated storage unit. The unillustrated storage unit may be provided in the exterior of the examination management device 12.

The examination information input unit 30 acquires, through the network 22, information about the insurance premium and the like that are sent from the insurance management device 14 and information about a specimen examination demand and the like that are sent from the examinee terminal device 18. Further, the examination information input unit 30 acquires information about a specimen examination result and the like that are sent from the examination organization terminal device 16.

The examination information output unit 32 outputs information about a calculation request for the insurance premium and the like to the insurance management device 14, through the network 22. The examination information output unit 32 outputs information about the examination result and the like to the examinee terminal device 18, through the network 22. The examination information output unit 32 outputs the examination result of the specimen examination, insurance information relevant to the insurance, and delivery information about the specimen examination kit.

In the present specification, data, information, a signal indicating data, and a signal indicating information can be mutually replaced. Here, the signal indicating data and the signal indicating information are also referred to as merely signals.

Further, as for the data, the information, and the signals and the like, acquisition, receiving, and input can be mutually replaced. Further, as for the data, the information, and the signals and the like, sending and output can be mutually replaced.

The examination analysis unit 34 analyzes the result of the specimen examination that is sent from the examination organization terminal device 16. As an example of the analysis of the examination result, there is derivation of the health degree based on the specimen examination result.

The examination plan unit 36 manages the schedule of the specimen examination that is acquired through the examination information input unit 30. As the schedule of the specimen examination, there are a delivery schedule of the specimen examination kit and an examination schedule.

The examination database 38 stores the specimen examination result for each examinee, in association with the examinee and the specimen examination result. In the examination database 38, the retrieval of the specimen examination can be performed, using the examinee as an index. The examination database 38 corresponds to an example of the examination result storage device.

### [Examination Organization Terminal Device]

The examination organization terminal device 16 is a terminal device that is used by an examiner. Herein, the examiner means an employee of the examination organization. The examiner may include an operator that inputs the examination result.

As the examination organization terminal device 16, a computer such as a personal computer, a tablet terminal device can be applied. As an example of the computer, there is a computer including a computer main body, an operation member and a display device. The same goes for the examinee terminal device 18, the insurance subscriber terminal device 19, and the insurance management terminal device 20 described later.

### [Insurance Management Device]

The insurance management device 14 includes an insurance management information input unit 50, an insurance management information output unit 52, a contract unit 54, and an insurance premium calculation unit 56. The insurance management device 14 is connected with the insurance management terminal device 20 and an insurance database 58, in a communicable manner. The connection form of the insurance management device 14 with the insurance management terminal device 20 and the insurance database 58 may be a connection using wires or a connection using no wire.

The disposition of the insurance management device 14 is the same as that of the examination management device 12. Further, the disposition of the insurance database 58 is the same as that of the examination database 38. Here, the description of the dispositions of the insurance management device 14 and the insurance database 58 is omitted.

The insurance management information input unit 50 acquires information about an insurance premium calculation request and the like that are sent from the examination management device 12 through the network 22. The insurance management information input unit 50 stores the acquired information in an unillustrated storage unit.

The insurance management information output unit 52 outputs a variety of information such as the insurance information that includes the insurance premium corresponding to the insurance premium calculation request and that is sent to the examination management device 12, through the network 22.

The contract unit 54 manages information relevant to the insurance contract. The contract unit 54 stores information about the contract condition and the like for each insurance contract, in the insurance database 58. The contract unit 54 reads information about the contract condition and the like for each insurance subscriber, by referring to the insurance database 58.

The insurance premium calculation unit 56 calculates the insurance premium based on the insurance premium calculation request. Further, the insurance premium calculation unit 56 stores information about the insurance premium and the like for each insurance contract, in the insurance database 58. The insurance premium calculation unit 56 can calculate the insurance premium based on the health degree, by referring to the insurance database 58 in which the relation between the health degree and the insurance premium is prescribed.

The insurance database 58 stores information about each insurance contract, for each insurance contract. The insurance database 58 is configured such that the insurance contract can be retrieved for each insurance contractor using the insurance contractor as an index. Further, the insurance database 58 is configured such that the insurance condition can be retrieved for each insurance contract using the insurance contract as an index.

The insurance database 58 stores the relation between the health degree and the insurance premium. The insurance database 58 may store the relation between the health degree and the insurance premium that is prescribed in consideration of the height, weight, BMI, and others of the insurance subscriber. The insurance database 58 may store the relation between the health degree and the insurance premium that is prescribed in consideration of taste information about whether the insurance subscriber is a smoker, whether the insurance subscriber is a drinker, and the like.

For example, when the BMI value is exceeding a reference value, the insurance premium may be increased. Further, for smokers and drinkers, the insurance premium may be increased. The relation between the health degree and the insurance premium may be stored in a database that is disposed in the exterior of the insurance management device 14. Here, the insurance management device 14 corresponds to an example of the insurance premium calculation device.

### [Insurance Management Terminal]

The insurance management terminal device 20 is a terminal device that is used by an insurance manager. As an example of the insurance manager, there is an employee of an insurance company or the like.

### [Receiver Terminal Device]

The examinee terminal device 18 is a terminal device that is used by an examinee that undergoes the specimen examination. The examinee terminal device 18 includes an unillustrated display device. The display device can display a variety of information that is acquired by the examinee terminal device 18. Here, the examinee that undergoes the specimen examination corresponds to an example of the specimen examination subject. The examinee terminal device 18 corresponds to an example of the subject terminal device.

### [Network]

As the network 22, a public communication line network may be applied, or a leased line network may be applied. To the network 22, a standardized specification may be applied, or a dedicated specification may be applied.

### [Hardware Configuration of Examination Management Device]

Figure 2 is a block diagram showing a configuration example of hardware of the examination management device. The examination management device 12 can realize the function of each unit of the above examination management device 12, by executing a program using the hardware shown in Figure 2.

The examination management device 12 includes a control unit 40, a memory 42, a storage device 44, a network controller 46, and a power supply device 48. The control unit 40, the memory 42, the storage device 44, and the network controller 46 are connected through a bus 49, such that data communication can be performed.

### <Control Unit>

The control unit 40 functions as a whole control unit, various arithmetic operation units, and a storage control unit of the examination management device 12. The control unit 40 executes programs stored in a ROM (read only memory) equipped in the memory 42.

The control unit 40 downloads a program from an external storage device through the network controller 46, and executes the downloaded program. The external storage device may be connected with the examination management device 12 through the network 22 in a communicable manner.

The control unit 40 uses a RAM (random access memory) equipped in the memory 42, as an arithmetic operation area, and executes various processes in cooperation with various programs. Thereby, various functions of the examination management device 12 are realized.

The control unit 40 controls reading of data from the storage device 44 and writing of data to the storage device 44. The control unit 40 may acquire a variety of data from the external storage device through the network controller 46. The control unit 40 can execute various processes such as arithmetic operation, using a variety of acquired data.

The control unit 40 may include one processor or two or more processors. As an example of the processor, there are an FPGA (Field Programmable Gate Array), a PLD (Programmable Logic Device) or the like. The FPGA and the PLD allow alteration of a circuit configuration after production.

As another example of the processor, there is an ASIC (Application Specific Integrated Circuit). The ASIC includes a circuit configuration that is specially designed for executing a specific process.

As the control unit 40, two or more processors that are the same in type can be applied. For example, as the control unit 40, two or more FPGAs may be used, or two PLDs may be used. As the control unit 40, two or more processors that are different in type may be applied. For example, as the control unit 40, one or more FPGAs and one or more ASICs may be applied.

In the case of including a plurality of control units 40, the plurality of control units 40 may be configured using one processor. As an example in which the plurality of control units 40 is configured by one processor, there is a form in which the one processor is configured using a combination of one or more CPUs (Central Processing Unit) and software and this processor functions as the plurality of control units 40. A GPU (Graphics Processing Unit) that is a processor dedicated to image processing may be applied, instead of the CPUs or in addition to the CPUs. Herein, the software is synonymous with a program. As a typical example in which the plurality of control units 40 is configured using one processor, there is a computer such as a client device and a server device.

As another example in which the plurality of control units 40 is configured by one processor, there is a form of using a processor that realizes the functions of the whole of a system including the plurality of control units 40 with one IC chip. As a typical example of the processor that realizes the functions of the whole of the system including the plurality of control units 40 with one IC chip, there is an SoC (System On Chip). Here, IC is an abbreviation of Integrated Circuit.

In this way, the control unit 40 is configured using one or more various processors, as a hardware structure.

### <Memory>

The memory 42 includes an unillustrated ROM and an unillustrated RAM The ROM stores various programs that are executed in the examination management device 12. The ROM stores parameters, files and others that are used for execution of various programs. The RAM functions as a temporary storage area for data, a work area for the control unit 40, and the like.

### <Storage Device>

The storage device 44 stores a variety of data in a non-transitory manner. The storage device 44 may be externally attached in the exterior of the examination management device 12. A high-capacity semiconductor memory device may be applied instead of the storage device 44 or in addition to this.

### <Network Controller>

The network controller 46 controls data communication with external devices such as the insurance management device 14, the examinee terminal device 18, and the examination organization terminal device 16 shown in Figure 1. The control of the data communication may include management of traffic of the data communication.

### <Power Supply Device>

As the power supply device 48, a high-capacity type power supply device such as a UPS (Uninterruptible Power Supply) is applied. When a commercial power supply is shut off due to a blackout or the like, the power supply device 48 supplies power to the examination management device 12. Note that the hardware configuration of the examination management device 12 shown in Figure 2 is an example and addition, removal, and alteration can be made when appropriate.

### [Function of Examination Management Device]

Figure 3 is a functional block diagram of the examination management device. The examination management device 12 shown in Figure 3 includes the examination information input unit 30, the examination information output unit 32, the examination analysis unit 34, and the examination plan unit 36. Details of each unit are described below.

### <Examination Information Acquisition Unit>

The examination information input unit 30 includes an examination result input unit 30A, an insurance information input unit 30B, and an examination acceptance information input unit 30C. The examination information input unit 30 may include various information acquisition units that acquire a variety of information. The illustration of the various information acquisition units is omitted.

As the examination result input unit 30A, the insurance information input unit 30B and the examination acceptance information input unit 30C, individual hardware may be applied, or common hardware may be applied. As a configuration of hardware of the examination information input unit 30, there is a configuration of including a signal input element, a signal conversion element and the like.

The examination result input unit 30A retrieves the examination result for each examinee and acquires the examination result for each examinee, from the examination database 38 shown in Figure 1. The examination result input unit 30A sends the acquired examination result to the examination analysis unit 34. The examination result input unit 30A corresponds to an example of the examination result acquisition unit.

The insurance information input unit 30B acquires the insurance information that is sent from the insurance management device 14. The insurance information input unit 30B sends the acquired insurance information to the examination analysis unit 34. The insurance information input unit 30B corresponds to an example of the insurance information acquisition unit.

The examination acceptance information input unit 30C acquires the examination acceptance information. The examination acceptance information input unit 30C sends the acquired examination acceptance information to the examination plan unit 36.

### <Examination Information Output Unit>

The examination information output unit 32 includes an examination result output unit 32A, an insurance information output unit 32B, a delivery information output unit 32C, and a health degree information output unit 32D. The examination information output unit 32 may include various information output units that output a variety of information. The illustration of the various information output units is omitted.

In the examination information output unit 32, as the examination result output unit 32A, the insurance information output unit 32B, the delivery information output unit 32C, and the health degree information output unit 32D, individual hardware may be applied, or common hardware may be applied. As a configuration of hardware of the examination information output unit 32, there is a configuration of including a signal output element, a signal conversion element and the like.

The examination result output unit 32A sends the examination result to the examinee terminal device 18. The insurance information output unit 32B sends the insurance information to the examinee terminal device 18.

The delivery information output unit 32C sends delivery information about the specimen examination kit to a delivery source of the specimen examination kit, based on a delivery instruction of the specimen examination kit that is sent from the examination plan unit 36. As the delivery source of the specimen examination kit, a specimen examination kit providing organization such as a specimen examination kit delivery section of the examination organization and a specimen examination kit delivery organization in the exterior of the examination organization can be applied. The health degree information output unit 32D sends health degree information including the heath degree, to the insurance management device 14.

The examination analysis unit 34 analyzes the specimen examination result that is acquired using the examination result output unit 32A, and derives the health degree of the examinee. The health degree is an index indicating the health state related to the insurance subscription of the examinee. As an example of the health degree, there is an age difference resulting from subtracting an actual age that is the real age of the examinee from a health state age of the examinee.

The health state age of the examinee is an age into which the health state of the examinee is converted. The health state age of the examinee is derived based on the result of the specimen examination of the examinee. The health state age of the examinee can be derived from the result of the blood examination of the examinee. HbA1c, e-FGR, ALB, AST, LDL cholesterol, ALP, and total cholesterol tend to vary depending on the age. From these examination item values, the health state age of the examinee can be derived.

For example, when the health state age of the examinee is derived from these examination item values, it is allowable to refer to a look-up table that specifies the relation of the above blood examination item values and the age, or to use a function that specifies the relation of the above blood examination item values and the age.

HbAlc represents glycohemoglobin. e-GFR represents estimated glomerular filtration rate. ALB represents albumin. AST represents aspartate aminotransferase. LDL is an abbreviation of low-density lipoprotein that means low specific gravity lipoprotein. ALP represents alkaline phosphatase.

HbAlc and e-FGR can be applied regardless of gender. ALB can be applied to males. AST, LDL cholesterol, ALP, and total cholesterol can be applied to females. LDL cholesterol corresponds to low-density lipoprotein cholesterol. The examination analysis unit 34 corresponds to an example of the health degree computation unit.

The examination plan unit 36 manages the schedule of the specimen examination in collaboration with the examination analysis unit 34. In the case of falling behind the schedule of the specimen examination, the examination plan unit 36 may send a warning using the examination information output unit 32.

### [Connection Example of Insurance Management Device and Insurance Subscriber Terminal]

Figure 4 is a block diagram showing a connection example of an insurance management device and an insurance subscriber terminal. The insurance management device 14 shown in Figure 4 is configured to be capable of being connected with the insurance subscriber terminal device 19 through the network 22.

The insurance subscriber terminal device 19 can access the insurance management device 14 through the network 22, can retrieve information about the insurance subscriber that is stored in the insurance database 58, and can acquire the information about the insurance subscriber.

### [Sequence of Specimen Examination Management Method According to First Embodiment]

Figure 5 is a flowchart showing a flow of a sequence of a specimen examination management method according to the first embodiment. In an examination acceptance step S10, the examination acceptance information input unit 30C shown in Figure 3 acquires the examination acceptance information that is sent from the examinee terminal device 18. In the examination acceptance step S10, the examination plan unit 36 registers the examinee based on the examination acceptance information. In the examination acceptance step S10, the examination plan unit 36 stores the examination condition in association with the examinee.

As the examination condition, there are the kind of the examination, the number of examinations, the frequency of the examination, and the like. In the examination acceptance step S10, the examination plan unit 36 may acquire, through the examination acceptance information input unit 30C, examinee information relevant to the examinee, as exemplified by the height, weight, and BMI of the examinee, whether the examinee is a smoker, and whether the examinee is a drinker. After the examination acceptance step S10, the flow proceeds to an examination result acquisition step S12.

In the examination result acquisition step S12, the examination result input unit 30A acquires the examination result that is sent from the examination organization terminal device 16. The examination result acquisition step S12 may be executed just before an insurance proposition sending step S24 or just after the insurance proposition sending step S24. The examination result is stored in the examination database 38. After the examination result acquisition step S12, the flow proceeds to a result sending step S14.

In the result sending step S14, the examination result output unit 32A sends the examination result to the examinee terminal device 18. After the result sending step S14, the flow proceeds to a result analysis step S16.

In the result analysis step S16, the examination analysis unit 34 derives the health degree based on the examination result. The derived health degree is stored in the examination database 38, in association with the examination result. After the result analysis step S16, the flow proceeds to an insurance premium calculation request sending step S18. The result analysis step S16 corresponds to an example of the health degree computation step.

In the insurance premium calculation request sending step S18, the insurance information output unit 32B sends an insurance premium calculation request to the insurance management device 14. The insurance premium calculation request includes the examinee information and the health degree of the examinee. After the insurance premium calculation request sending step S18, the flow proceeds to an insurance premium calculation result acquisition step S20.

In the insurance premium calculation result acquisition step S20, the insurance information input unit 30B acquires the insurance premium calculation result that is sent from the insurance management device 14. After the insurance premium calculation result acquisition step S20, the flow proceeds to an insurance proposition creation step S22. The insurance premium calculation result acquisition step S20 corresponds to an example of the insurance information acquisition step of acquiring the insurance information including the insurance premium based on the health degree of the specimen examination subject.

In the insurance proposition creation step S22, the examination analysis unit 34 creates the insurance proposition based on the insurance premium calculation result. After the insurance proposition creation step S22, the flow proceeds to the insurance proposition sending step S24. The examination analysis unit 34 corresponds to an example of the insurance proposition creation unit configured to create the insurance proposition.

In the insurance proposition sending step S24, the insurance information output unit 32B sends the insurance proposition to the examinee terminal device 18. After the insurance proposition sending step S24, the flow proceeds to an insurance application determination step S26. The insurance information output unit 32B corresponds to an example of the insurance proposition output unit configured to output the insurance proposition. The insurance proposition sending step S24 corresponds to an example of the insurance proposition output step of outputting the insurance proposition.

In the insurance application determination step S26, it is determined whether the examination plan unit 36 has acquired the insurance application that is sent from the examinee terminal device 18. In the insurance application determination step S26, in the case where the examination plan unit 36 has not acquired the insurance application, the No determination is made. In the case of the No determination, the examination management device 12 ends the specimen examination management method.

On the other hand, in the insurance application determination step S26, in the case where the examination plan unit 36 has acquired the insurance application, the Yes determination is made. In the case of the Yes determination, the flow proceeds to an insurance application acceptance step S28.

In the insurance application acceptance step S28, the examination analysis unit 34 accepts the insurance application that is sent from the examinee terminal device 18. In the insurance application acceptance step S28, the examination analysis unit 34 sends the insurance application to the insurance management device 14 through the insurance information output unit 32B. After the insurance application acceptance step S28, the flow proceeds to a completion notice sending step S30.

In the completion notice sending step S30, the examination plan unit 36 sends, to the examinee terminal device 18, a completion notice indicating that the insurance application has been sent to the insurance management device 14. After the completion notice sending step S30, the examination management device 12 ends the specimen examination management method.

Figure 6 is a flowchart showing a flow of information among constituent elements of the specimen examination management system and a flow of the blood examination kit. In an examination application step S100, an examination application is sent from the examinee terminal device 18 to the examination management device 12.

In an examination acceptance step S102, the examination management device 12 accepts the examination application that is sent from the examinee terminal device 18. The examination acceptance step S102 corresponds to the examination acceptance step S10 shown in Figure 5.

In a registration step S104, the examination management device 12 stores information relevant to the examinee. In the registration step S104, the examination management device 12 may give an identification number for each examinee, to the examinee.

In a kit delivery step S106, the examination management device 12 requests the examination organization to deliver the specimen examination kit to a prescribed delivery address, based on the delivery information about the specimen examination kit. The examination organization delivers the specimen examination kit to the prescribed delivery address. The delivery of the specimen examination kit may be executed by a delivery company that has received a request from the examination organization.

In a kit receiving step S108, the examinee acquires the specimen examination kit. The examinee can acquire the specimen examination kit at the home, a drug store, a store other than the drug store, a service office of the delivery company, a post office or the like.

In an examination taking step S110, the examinee or the like takes the specimen of the examinee, using the specimen examination kit. In the specimen delivery step S112, the examinee or the like delivers the taken specimen to the examination organization.

In the examination step S114, the examination organization performs the examination of the specimen that is delivered from the examinee. The examination organization terminal device 16 sends the examination result to the examination management device 12. The examination management device 12 receives the examination result that is sent from the examination organization terminal device 16. The step in which the examination management device 12 receives the examination result corresponds to the examination result acquisition step S12 shown in Figure 5.

In a result sending step S 116, the examination management device 12 sends the examination result to the examinee terminal device 18. The result sending step S 116 corresponds to the result sending step S14 shown in Figure 5. In an examination result receiving step S 118, the examinee terminal device 18 receives the examination result. In a result analysis step S120, the examination management device 12 analyzes the examination result, and derives the health degree of the examinee. The result analysis step S120 corresponds to the result analysis step S16 shown in Figure 5.

In an insurance premium calculation request sending step S122, the examination management device 12 sends the insurance premium calculation request to the insurance management device 14. The insurance premium calculation request sending step S122 corresponds to the insurance premium calculation request sending step S18 shown in Figure 5.

In an insurance premium calculation request receiving step S124, the insurance management device 14 receives the insurance premium calculation request that is sent from the examination management device 12. In an insurance premium calculation step S126, the insurance management device 14 calculates the insurance premium based on the health degree of the examinee. In the calculation of the insurance premium, it is possible to refer to the insurance database 58 in which the relation of the health degree and the insurance premium is prescribed.

In an insurance premium calculation result sending step S128, the insurance management device 14 sends the insurance premium calculation result to the examination management device 12. In an insurance premium calculation result receiving step S130, the examination management device 12 receives the insurance premium calculation result that is sent from the insurance management device 14. The insurance premium calculation result receiving step S130 corresponds to the insurance premium calculation result acquisition step S20 shown in Figure 5.

In an insurance proposition creation step S132, the examination management device 12 creates the insurance proposition based on the insurance premium calculation result that is sent from the insurance management device 14. The insurance proposition creation step S132 corresponds to the insurance proposition creation step S22 shown in Figure 5.

In an insurance proposition sending step S134, the examination management device 12 sends the insurance proposition to the examinee terminal device 18. The insurance proposition sending step S134 corresponds to the insurance proposition sending step S24 shown in Figure 5.

In an insurance proposition receiving step S136, the examinee terminal device 18 receives the insurance proposition that is sent from the examination management device 12. In an insurance subscription determination step S138, in the case where the examinee does not hope for the insurance subscription, the No determination is made. In the case of the No determination, the flow proceeds to a non-subscription information sending step S139. In the non-subscription information sending step S139, non-subscription information indicating that the examinee does not hope for the insurance subscription is sent from the examinee terminal device 18 to the examination management device 12.

Here, for the examination result receiving step S118 in which the examinee receives the examination result and the insurance proposition receiving step S136 in which the examinee receives the insurance proposition, the timings may coincide for concurrent execution. That is, the examinee terminal device 18 may receive the examination result and the insurance proposition concurrently. Herein, the concurrent execution can include a substantial concurrent execution that has a timing difference due to an error such as a variation in receiving timing and a variation in communication period.

In a non-subscription information receiving step S140, the examination management device 12 receives the non-subscription information that is sent from the examinee terminal device 18. After the non-subscription information receiving step S140, the examination management device 12 ends the examination management method in the specimen examination management system 10.

On the other hand, in the insurance subscription determination step S138, in the case where the examinee hopes for the insurance subscription, the Yes determination is made. In the case of the Yes determination, the flow proceeds to an insurance application sending step S141. In the insurance application sending step S141, the insurance application is sent from the examinee terminal device 18 to the examination management device 12.

In an insurance application receiving step S142, the examination management device 12 receives the insurance application that is sent from the examinee terminal device 18. The non-subscription information receiving step S140 and the insurance application receiving step S142 are included in the insurance application determination step S26 shown in Figure 5.

In an insurance application sending step S144, the insurance application of the examinee is sent from the examination management device 12 to the insurance management device 14. The insurance application sending step S144 corresponds to the insurance application acceptance step S28 shown in Figure 5.

In an insurance application receiving step S146, the insurance management device 14 receives the insurance application of the examinee that is sent from the examination management device 12. In a subscription procedure step S148, the insurance management device 14 performs an insurance subscription procedure for the examinee, based on the insurance application of the examinee. In the insurance subscription procedure, sending and receiving of information necessary for the subscription procedure may be executed between the insurance management device 14 and the examinee terminal device 18.

When the insurance management device 14 has completed the insurance subscription procedure for the examinee, the insurance management device 14 sends a completion notice indicating that the insurance subscription procedure has been completed, to the examination management device 12, in a completion notice sending step S150.

In a completion notice receiving step S152, the examination management device 12 receives the completion notice that is sent from the insurance management device 14. In a completion notice sending step S154, the examination management device 12 sends the completion notice to the examinee terminal device 18. The completion notice sending step S154 corresponds to the completion notice sending step S30 shown in Figure 5. After the completion notice sending step S154, the examination management device 12 ends the examination management method in the specimen examination management system 10.

In the completion notice receiving step S156, the examinee terminal device 18 receives the completion notice that is sent from the examination management device 12.

### [Screen Display of Examinee Terminal Device]

Next, a screen display that is displayed using a display unit equipped in the examinee terminal is described. Note that each screen display described below is an example and alteration, addition, and removal of constituent elements can be made.

### [Configuration Example of Examination Result Screen]

Figure 7 is an explanatory diagram showing an example of an examination result screen. An examination result screen 120 shown in Figure 7 is generated based on the examination result that is sent from the examination management device 12 to the examinee terminal device 18. The examination result screen 120 can be displayed using the display unit equipped in the examinee terminal device 18, after the examination result receiving step S118 shown in Figure 6.

The examination result screen 120 shown in Figure 7 includes demander information 102, examination date information 122, examination kit information 124, and examination result list 126.

The examination date information 122 may be a specimen taking date when the examinee takes the specimen, or may be an examination analysis date when the examination organization analyzes the specimen. The examination result screen 120 may display at least one of the specimen taking date and the specimen analysis date.

The examination kit information 124 is information that specifies the specimen kit delivered to the examinee. The examination kit information 124 shown in Figure 7 is the serial number of the specimen examination kit. As the examination kit information 124, the management number of the specimen examination kit, the production lot number of the specimen examination kit, or the like may be applied. Here, the alphabets illustrated in Figure 7 shows arbitrary numerical values or arbitrary alphabets. The same goes for Figure 8, Figure 9, Figure 14, and Figure 15.

On the examination result list 126, an item value of each specimen examination item is displayed. The examination result list 126 shown in Figure 7 has a list table format. The format of the examination result list 126 is not limited. For example, a graph format or the like may be applied. On the examination result list 126, all of the specimen examination items may be displayed, or some of the specimen examination items may be displayed.

On the examination result list 126 shown in Figure 7, blood glucose, HbA1c, HDL cholesterol, LDL cholesterol, neutral fat, AST, ALT, γ-GPT, ALP, ALB, and e-GFR are exemplified as blood examination items.

HDL is an abbreviation of high density lipoprotein that means high specific gravity lipoprotein. ALT represents alanine aminotransferase. γ-GPT represents gamma glutamyl transpeptidase. ALB represents albumin.

As examples of the examination item when the specimen is urine, there are urine protein, uric blood, urobilinogen and the like. As examples of the examination item when the specimen is stool, there are a dysentery bacillus, a salmonella, an intestinal hemorrhagic Escherichia coli, and the like. The salmonella can include at least one of Salmonella paratyphi A and Salmonella typhi. The intestinal hemorrhagic Escherichia coli can include at least one of 0157, O111, and O26.

Further, a reference range of each examination item may be added to the examination result, and with respect to the reference range of each examination item, a comment of whether the examination result is within the reference range or whether the examination result is beyond the reference range may be added. A display for encouraging an improvement when the examination result is beyond the reference range with respect to the reference range of each examination item may be added. As an example of the improvement, there is diet or the like.

### [Configuration Example of Insurance Proposition Screen]

Figure 8 is an explanatory diagram showing an example of an insurance proposition screen. An insurance proposition screen 140 shown in Figure 8 can be displayed using the display unit equipped in the examinee terminal device 18, based on the insurance proposition that is received in the insurance proposition receiving step S136 shown in Figure 6.

The insurance proposition screen 140 includes the demander information 102, health degree information 142, insurance premium information 144, and an insurance application selection button 146. As the health degree information 142 shown in Figure 8, the age difference resulting from subtracting the actual age from the health state age as the health degree is applied. As the health degree information 142, a rank of the health degree may be expressed in five levels of A to E, in which the highest rank is A and the lowest rank is E.

The insurance premium information 144 indicates information about the insurance premium that is calculated based on the health degree of the examinee. The insurance premium information 144 shown in Figure 8 indicates a discount rate of a basic insurance premium that is calculated from the actual age without considering the health degree. The insurance premium that is calculated based on the health degree of the examinee may be displayed as the insurance premium information 144. The insurance premium information 144 is a constituent element of the insurance information.

The insurance application selection button 146 is a button that is used in selection of whether to apply for the insurance. The insurance application selection button 146 allows selection of one of YES and NO. The selection information is sent from the examinee terminal device 18 to the examination management device 12.

### [Configuration Example of Application Completion Screen]

Figure 9 is an explanatory diagram showing an example of an application completion screen. An application completion screen 160 shown in Figure 9 can be displayed using the display unit equipped in the examinee terminal device 18, based on the completion notice that is sent from the examination management device 12 to the examinee terminal device 18 in the completion notice sending step S154 shown in Figure 6.

The application completion screen 160 includes the demander information 102, application completion information 162, and an OK button 164. As the application completion information 162 shown in Figure 9, character information indicating that the application of the insurance has been accepted is applied.

The OK button 164 is a button that is operated when the examinee closes the application completion screen 160. When the OK button 164 is clicked, the application completion screen 160 is closed.

### [Function Effect of First Embodiment]

With the specimen examination management device, system, and method according to the first embodiment configured as described above, it is possible to obtain the following function effects.
[1] The insurance premium based on the health degree of the examinee that indicates the health state related to the insurance subscription is acquired. Thereby, it is possible to handle the proposition of the insurance premium corresponding to the health consciousness of the examinee.
[2] The examinee terminal device 18 acquires the insurance proposition in which the insurance premium corresponding to the specimen examination result when the specimen examination is underwent is calculated. Thereby, when the examination is performed at the time of the insurance subscription, it is possible to avoid the opportunity of the examination from going to waste because the examination result is not good and the merit of the insurance premium reduction is not obtained.
[3] The examination management device 12 stores the examination result, and stores the insurance premium that is calculated based on the examination result, in association with the examination result. Thereby, the examination result is surely associated with the insurance premium. In the case of the application of the insurance with the self-report of the examination result, it is sometimes hard to know the time of the examination, the method of the examination and the like, and it is difficult to reflect, in the insurance premium, an exact actual state of a person that hopes for the insurance subscription.
[4] The examinee takes the specimen using the specimen examination kit that is delivered from the examination organization or the like. The examinee delivers the taken specimen to the examination organization. Thereby, the examinee can undergo the specimen examination without going to a hospital, the examination organization or the like.

### [Applying Example of Network System]

Figure 10 is a block diagram showing a configuration example of a specimen examination management system to which a network system is applied. In a specimen examination management system 10A shown in Figure 10, the examination management device 12, the insurance management device 14, the examination organization terminal device 16, the examinee terminal device 18, the insurance management terminal device 20, the examination database 38, and the insurance database 58 are connected through networks 22 in a communicable manner.

In Figure 10, a plurality of networks 22 is illustrated. The plurality of networks 22 shown in Figure 10 shows the network 22 shown in Figure 1. The constituent elements of the specimen examination management system 10A shown in Figure 10 are the same as the constituent elements of the specimen examination management system 10 shown in Figure 1. Here, the description of each constituent element of the specimen examination management system 10A is omitted.

As the specimen examination management system 10A, a distributed network system is applied, and the respective constituent elements of the specimen examination management system 10A are connected through the networks 22 in a communicable manner.

Thereby, the examination management device 12, the insurance management terminal device 20, and the examination database 38 can be disposed so as to be distributed. Similarly, the insurance management device 14, the insurance management terminal device 20, and the insurance database 58 can be disposed so as to be distributed.

For example, the examination organization may be in Japan, the examination organization terminal device 16 may be disposed in Japan, and the examination management device 12 and the examination database 38 may be disposed in a foreign country or the like. The foreign country or the like means a country or region other than Japan.

### [Modification of Specimen Examination Management System]

Figure 11 is a block diagram showing a configuration example of a specimen examination management system according to a modification. A specimen examination management system 10B shown in Figure 11 includes an examination insurance management integration device 11 in which the examination management device 12 and insurance management device 14 shown in Figure 1 are integrated.

The examination insurance management integration device 11 is connected with the insurance subscriber terminal device 19 through the network 22 in a communicable manner. The insurance subscriber terminal device 19 is a terminal device that is used by the insurance subscriber.

The examination insurance management integration device 11 includes an examination management unit 12B and an insurance management unit 14B. The examination management unit 12B has the same configuration and function as the examination management device 12 shown in Figure 1. The insurance management unit 14B has the same configuration and function as the insurance management device 14 shown in Figure 1.

Figure 11 exemplifies a mode in which the examination management unit 12B includes the examination database 38 and the insurance management unit 14B includes the insurance database 58, but the examination database 38 and the insurance database 58 may be disposed in the exterior of the examination insurance management integration device 11. Further, an integration database in which the examination database 38 and the insurance database 58 are integrated may be included.

### [Sequence of Specimen Examination Management Method According to Second Embodiment]

Figure 12 is a flowchart showing a flow of a sequence of a specimen examination management method according to a second embodiment. The specimen examination management method according to the second embodiment is applied in the case where the specimen examination is performed when the insurance subscriber having already subscribed the insurance continues the insurance.

Note that the same configuration as the specimen examination management system 10 according to the first embodiment can be applied to the specimen examination management method according to the second embodiment. Here, the illustration and description of the specimen examination management system according to the second embodiment are omitted.

In the specimen examination management method according to the second embodiment, an insurance premium increase-reduction determination step S200, a re-examination necessity sending step S202, and a re-examination request reception determination step S204 shown in Figure 12 are added to the specimen examination management method according to the first embodiment shown in the flowchart of Figure 5.

As shown in Figure 12, after the insurance premium calculation result acquisition step S20, the flow proceeds to the insurance premium increase-reduction determination step S200. In the insurance premium increase-reduction determination step S200, the examination analysis unit 34 shown in Figure 3 determines whether the insurance premium of the insurance after the continuation is equal to or more than the insurance premium of the current insurance.

In the insurance premium increase-reduction determination step S200, in the case where the examination analysis unit 34 determines that the insurance premium is reduced or is equal, the No determination is made. In the case of the No determination, the flow proceeds to the insurance proposition creation step S22.

On the other hand, in the insurance premium increase-reduction determination step S200, in the case where the examination analysis unit 34 determines that the insurance premium is increased, the Yes determination is made. In the case of the Yes determination, the flow proceeds to the re-examination necessity sending step S202.

In the re-examination necessity sending step S202, the examination analysis unit 34 sends necessity confirmation information for confirming the necessity of re-examination, to the examinee terminal device 18 through the examination information output unit 32. After the re-examination necessity sending step S202, the flow proceeds to the re-examination request reception determination step S204.

In the re-examination request reception determination step S204, it is determined whether necessity information indicating the necessity of the re-examination has been received. In the re-examination request reception determination step S204, in the case where the necessity information has not been received or in the case where the necessity information indicating that the re-examination is unnecessary has been received, the No determination is made. In the case of the No determination, the flow proceeds to the insurance proposition creation step S22.

On the other hand, in the re-examination request reception determination step S204, in the case where the necessity information indicating that the re-examination is necessary has been received, the Yes determination is made. In the case of the Yes determination, the flow proceeds to the examination acceptance step S10 shown in Figure 5.

In the examination management method according to the second embodiment, the number of re-examinations may be restricted. For example, after the insurance premium increase-reduction determination step S200, a re-examination number determination step of determining whether the number of re-examinations is equal to or less than a prescribed number can be added.

In the case where the number of re-examinations is exceeding the prescribed number, a warning step can be executed. In the warning step, the examination analysis unit 34 can send a warning indicating that the number of re-examinations is exceeding the prescribed number, to the examinee terminal device 18. After the warning step, the flow can proceed to the insurance proposition creation step S22. On the other hand, in the case where the number of re-examinations is equal to or less than the prescribed number, the flow can proceed to the examination acceptance step S10.

Figure 13 is a flowchart showing a flow of information among constituent elements of the specimen examination management system and a flow of the blood examination kit in the specimen examination management method according to the second embodiment. First, in an expiration notice sending step S220, an expiration notice is sent from the insurance management device 14 to the examinee terminal device 18. The expiration notice is sent from the insurance management device 14 to the examinee terminal device 18, during an insurance contract continuation procedure allowing period in which a continuation procedure of the insurance contract is allowed.

The expiration notice is sent from the insurance management device 14 to the examinee terminal device 18, until a prescribed period before the date of the insurance expiration during the insurance contract continuation procedure allowing period. The display unit of the examinee terminal device 18 may display the expiration notice. For example, the prescribed period may be two months. The expiration notice may be performed one month before the date of the insurance expiration at the latest.

Here, the expiration notice corresponds to an example of the intention confirmation information for confirming the continuation intention for the insurance contract. The insurance information output unit 32B of the examination management device 12 corresponds to an example of the intention confirmation information output unit configured to output the intention confirmation information to the subject terminal device.

An expiration notice sending step S221 of sending the expiration notice from the examination management device 12 to the examinee terminal device 18 may be executed instead of the expiration notice sending step S220.

In an expiration notice receiving step S222, the examinee terminal device 18 receives the expiration notice that is sent from the insurance management device 14.

In a continuation hope information sending step S224, in the case where the insurance subscriber hopes for the insurance continuation, the insurance subscriber sends continuation hope information indicating that the insurance subscriber hopes for the insurance continuation, from the examinee terminal device 18 to the examination management device 12. The insurance subscriber may send the continuation hope information from the examinee terminal device 18 to the insurance management device 14, and the insurance management device 14 may transfer the continuation hope information to the examination management device 12.

In a continuation hope information receiving step S226, the examination management device 12 receives the continuation hope information that is sent from the examinee terminal device 18. The continuation hope information corresponds to an example of the intention information indicating the continuation intention for the insurance contract of the specimen examination subject. The insurance information input unit 30B of the examination management device 12 corresponds to an example of the intention information acquisition unit configured to acquire the intention information. In the continuation hope information receiving step S226, the continuation hope information may be transferred from the examination management device 12 to the insurance management device 14.

In a registration step S228, the examination management device 12 refers to the examination database 38, and registers the continuation hope information. After the registration step S228, the respective steps from the kit delivery step S106 to the insurance proposition receiving step S136 are executed.

The respective steps from the kit delivery step S106 to the insurance proposition receiving step S136 are the same as those in Figure 6. Here, the description is omitted. However, the insurance premium increase-reduction determination step S200 to the re-examination request reception determination step S204 shown in Figure 12 are executed between the insurance premium calculation result receiving step S130 and the insurance proposition creation step S132.

After the insurance proposition receiving step S136, the flow proceeds to an insurance continuation determination step S230. In the insurance continuation determination step S230, in the case where the insurance subscriber does not hope for the insurance continuation, the No determination is made. In the case of the No determination, the flow proceeds to a non-continuation information sending step S232. In the non-continuation information sending step S232, the insurance subscriber sends non-continuation information indicating that the insurance subscriber does not hope for the insurance continuation, from the examinee terminal device 18 to the examination management device 12.

In a non-continuation information receiving step S234, the examination management device 12 receives the non-continuation information that is sent from the examinee terminal device 18. After the non-continuation information receiving step S234, the examination management device 12 ends the examination management method in the specimen examination management system 10.

On the other hand, in the insurance continuation determination step S230, in the case where the examinee hopes for the insurance continuation, the Yes determination is made. In the case of the Yes determination, the flow proceeds to a continuation application sending step S236. In the continuation application sending step S236, a continuation application is sent from the examinee terminal device 18 to the examination management device 12.

In an insurance continuation receiving step S238, the examination management device 12 receives the continuation application that is sent from the examinee terminal device 18. In an insurance continuation sending step S240, the examination management device 12 sends the continuation application to the insurance management device 14.

In an insurance continuation undergoing step S242, the insurance management device 14 receives the continuation application that is sent from the examination management device 12.

In a continuation procedure step S244, the insurance management device 14 performs the continuation procedure of the insurance subscriber, based on the continuation application of the insurance subscriber. After the continuation procedure step S244, the flow proceeds to the completion notice sending step S150. Subsequently, the respective steps from the completion notice sending step S150 to the completion notice receiving step S156 are executed. The respective steps from the completion notice sending step S150 to the completion notice receiving step S156 are the same as those in Figure 6. Here, the description thereof is omitted.

### [Screen Display of Examinee Terminal Device]

### [Configuration Example of Insurance Proposition Screen Including Re-examination Acceptance]

Figure 14 is an explanatory diagram showing an example of an insurance proposition screen including a re-examination acceptance. An insurance proposition screen 200 shown in Figure 14 can be displayed using the display unit equipped in the examinee terminal device 18, based on the insurance proposition that is received in the insurance proposition receiving step S136 shown in Figure 13.

The insurance proposition screen 200 includes a re-examination hope selection button 206 for selecting whether to hope for the re-examination, instead of the insurance application selection button 146 shown in Figure 8. The health degree information 142 shown in Figure 14 indicates that the health state age is the actual age plus 5. Further, the insurance premium information 144 indicates that the insurance premium is increased by 10 percent.

The re-examination hope selection button 206 is a button that is used in selection of whether to hope for the re-examination. The re-examination hope selection button 206 allows selection of one of YES and NO. The selection information is sent from the examinee terminal device 18 to the examination management device 12.

### [Configuration Example of Re-examination Acceptance Completion Screen]

Figure 15 is an explanatory diagram showing an example of a re-examination acceptance completion screen. A re-examination acceptance completion screen 220 shown in Figure 15 can be displayed using the display unit equipped in the examinee terminal device 18, based on the completion notice of the re-examination that is sent from the examination management device 12 to the examinee terminal device 18 in the completion notice sending step S154 shown in Figure 13.

The re-examination acceptance completion screen 220 includes a demander information 102, a re-examination application completion information 222, and an OK button 224. As the re-examination application completion information 222 shown in Figure 15, character information indicating that the application of the re-examination has been accepted is applied.

The OK button 224 is a button that is operated when the examinee closes the re-examination acceptance completion screen 220. When the OK button 224 is clicked, the re-examination acceptance completion screen 220 is closed.

### [Application Example of Second Embodiment]

### [Example of Review of Insurance Condition]

In the case where the insurance premium of the insurance after the continuation is equal to or more than the insurance premium of the insurance under contract, review of the insurance condition is proposed. Thereby, it is possible to avoid the increase in the insurance premium. The review of the insurance condition includes at least one of shortening of the insurance period and reduction of security.

Figure 16 is a flowchart showing a flow of a specimen examination management method according to an application example of the second embodiment. In the specimen examination management method according to the application example of the second embodiment, in the case of the Yes determination in the insurance premium increase-reduction determination step S200, the flow proceeds to an insurance condition review necessity sending step S210.

In the insurance condition review necessity sending step S210, the examination analysis unit 34 sends, to the examinee terminal device 18, increase information indicating that the insurance premium is increased relative to the insurance premium of the current insurance, and a selection information sending request for requesting the sending of selection information indicating whether to review the insurance condition. After the insurance condition review necessity sending step S210, the flow proceeds to a review request reception determination step S212.

In the review request reception determination step S212, the examination analysis unit 34 determines whether the selection information sent from the examinee terminal device 18 indicates that the insurance condition is reviewed or that the insurance condition is not reviewed. In the review request reception determination step S212, in the case where the examination analysis unit 34 determines that the examination analysis unit 34 has received the selection information indicating that the insurance condition is not reviewed, the No determination is made. In the case of the No determination, the flow proceeds to the insurance proposition creation step S22. In the insurance proposition creation step S22, the examination management device 12 creates the insurance proposition in which the insurance condition is maintained.

On the other hand, in the review request reception determination step S212, in the case where it is determined that the examination management device 12 has received the selection information indicating that the insurance condition is reviewed, the Yes determination is made. In the case of the Yes determination, the flow proceeds to an insurance condition review step S214.

In the insurance condition review step S214, the insurance premium calculation request when the insurance condition is reviewed is sent from the examination management device 12 to the insurance management device 14. That is, in the insurance condition review step S214, the examination management device 12 makes an inquiry to the insurance management device 14 about whether there is an insurance condition in which the insurance premium is not increased.

In the case where there is no insurance condition in which the insurance premium is not increased, the insurance management device 14 sends information indicating that effect, to the examination management device 12. On the other hand, in the case where there is an insurance condition in which the insurance premium is not increased, the insurance management device 14 sends, to the examination management device 12, the insurance condition in which the insurance premium is not increased and the insurance premium. After the insurance condition review step S214, the flow proceeds to the insurance proposition creation step S22.

In the insurance proposition creation step S22, the examination management device 12 creates the insurance proposition based on the information that is sent from the insurance management device 14.

### [Configuration Example of Insurance Proposition Screen Including Insurance Condition Review Selection]

Figure 17 is an explanatory diagram showing an example of an insurance proposition screen including an insurance condition review selection. An insurance proposition screen 240 shown in Figure 17 includes the health degree information 142, the insurance premium information 144, an insurance condition review selection button 246, and a condition selection button 248.

The health degree information 142 and insurance premium information 144 shown in Figure 17 are the same as those in Figure 14. Here, the description is omitted.

The insurance condition review selection button 246 allows selection of one of YES and NO. The selection information is sent from the examinee terminal device 18 to the examination management device 12.

The condition selection button 248 allows selection of one of period shortening and security reduction. The selection information is sent from the examinee terminal device 18 to the examination management device 12.

### [Configuration Example of Insurance Condition Review Acceptance Completion Screen]

Figure 18 is an explanatory diagram showing an example of an insurance condition review acceptance completion screen. An insurance condition review acceptance completion screen 260 shown in Figure 18 can be displayed using the display unit equipped in the examinee terminal device 18, based on the completion notice that is sent from the examination management device 12 to the examinee terminal device 18.

On the insurance condition review acceptance completion screen 260, character information indicating that the review of the security has been accepted is applied as application completion information 262 indicating that the review of the security is performed.

An OK button 264 is a button that is operated when the examinee closes the insurance condition review acceptance completion screen 260. When the OK button 264 is clicked, the insurance condition review acceptance completion screen 260 is closed.

As an example of the review of the insurance condition, there is shortening of the insurance period. For example, in the case where the current insurance period is one year, the insurance period after the continuation is shortened to half year, and the specimen examination is performed at the end of the insurance period of the insurance after the continuation. As another example of the review of the insurance condition, there is reduction of the security. For example, in the life insurance, there are reduction of insurance amount, termination of an option contract such as medical security.

### [Function Effect of Second Embodiment]

With the specimen examination management device, system, and method according to the second embodiment configured as described above, it is possible to obtain the following function effects.
[1] The specimen examination is performed at the time of the insurance continuation. It is possible to select the execution of the re-examination, in the case where the insurance premium calculated using the health degree based on the specimen examination result is more than the current insurance premium. Thereby, it is possible to execute the re-examination in the case where the timing of the specimen examination is bad.
[2] The health degree is calculated depending on the result of the re-examination. Thereby, it is possible to avoid the increase in the insurance premium in the case where the result of the re-examination is good.
[3] It is possible to select the review of the insurance condition in the case where the insurance premium of the insurance after the continuation is more than the current insurance premium. Thereby, it is possible to avoid the insurance premium of the insurance after the continuation from being more than the current insurance premium.

### [Applying Example of Personal Authentication Information]

The examination management device 12 shown in Figure 1 can store personal authentication information about the specimen examination subject in the examination database 38, in association with the result of the specimen examination. The personal authentication information is information for authenticating the result of the specimen examination stored in the examination database 38 as the result for the specimen examination subject oneself.

That is, the examination management device 12 includes an unillustrated personal authentication information acquisition unit. The examination analysis unit 34 associates the personal authentication information for each specimen examination subject that is acquired using the personal authentication information acquisition unit, with the information of the result of the specimen examination. The result of the specimen examination that is associated with the personal authentication information is stored in the examination database 38.

A known technique can be applied to the personal authentication. As an example of a known personal authentication, there is a technique of collating personal information previously registered and personal information sent from the examinee terminal device 18.

As an example of the personal information sent from the examinee terminal device 18, there is pick up data that is obtained by picking up the person in question, an identification certificate or the like using a pickup device such as a camera. As the camera, a camera included in the examinee terminal device 18 can be applied. As the camera, a camera that is connected with the examinee terminal device 18 can be applied. The camera only needs to have one function of still image pickup and moving image pickup. As the identification certificate, a previously issued identification certificate for the specimen examination management system 10 may be applied. An existing identification certificate such as a driver license and a passport may be applied.

The authenticated result of the specimen examination to which the personal authentication information is added can be used as contract assessment information for determining whether the insurance subscription, insurance renewal or the like is permitted. Further, in the case of obtaining a medical measurement value using a medical measurement apparatus, for example, a blood pressure measurement value using a simplified blood pressure measurement apparatus that is operated by a measurer oneself, the medical measurement value can be used for determining whether the insurance subscription, insurance renewal or the like is permitted.

That is, the examination management device 12 includes an unillustrated medical measurement value acquisition unit. The examination analysis unit 34 associates the medical measurement value acquisition unit for each specimen examination subject that is acquired using the medical measurement value acquisition unit, with the result of information about the specimen examination. The result of the specimen examination that is associated with the medical measurement value acquisition unit is stored in the examination database 38.

### [Specific Example of Specimen Examination Kit]

### [Blood Examination Kit]

Figure 19 is a configuration diagram of the blood examination kit. A blood examination kit 500 shown in Figure 19 is a specimen examination kit that makes it possible to take human blood as the specimen. The blood examination kit 500 shown in Figure 19 includes a sealing cap 502, a cylinder 504, an aspirator 506, a lancet 508, and a bottle 510 containing a dilution buffer solution.

The sealing cap 502, the cylinder 504, the aspirator 506, the lancet 508, and the bottle 510 are put in a case 514. The blood examination kit 500 may include an unillustrated adhesive plaster and sterile cloth.

### [Sequence of Blood Taking Using Blood Examination Kit]

For taking blood using the blood examination kit 500, the following sequence is applied. First, the pulp of a finger is caused to bleed using the lancet 508, and the blood on the pulp of the finger is absorbed by the whole of an absorber 512 at a tip of the aspirator 506. As the absorber 512, an inner cotton can be applied. Next, the aspirator 506 is inserted into the bottle 510 containing the dilution buffer solution, and the absorber 512 is dropped in the dilution buffer solution within the bottle 510. In the way, the blood in the absorber 512 is dispersed in the dilution buffer solution.

Next, the cylinder 504 is inserted into the bottle 510, the cylinder 504 is pushed to the bottom of the bottle 510, and the blood dispersed in the dilution buffer solution is separated into blood cells and blood plasma using a separation filter. In this state, the sealing cap 502 is attached to the bottle 510. The bottle 510 with the sealing cap 502 is packed using a dedicated packing material, and is delivered to a blood examination organization.

### [Function Effect of Blood Taking Using Blood Examination Kit]

According to the blood taking using the blood examination kit 500 described above, it is possible to obtain the following function effects.
[1] It is possible to perform the blood taking at a place other than a medical organization, as exemplified by the home.
[2] The blood examination kit is delivered on an examination date prescribed in a specimen examination plan, and therefore it is possible to prevent a failure of the blood taking, an error of the blood taking date, and the like.
[3] The blood taking amount is as small as 0.1 milliliters or less.

### [Applying Example of Specimen Examination Plan Management to Animal Other Than Human]

### [Description of Problem]

Pet owners having pets are increasing every year. An increasing number of pet owners regard pets as family members, and take care of health management of pets similarly to human. Currently, there is no public medical security system for pets, and pet owners subscribe to arbitrary pet insurance policies to reduce medical care costs for pets.

Although the health management of pets increases, setting of insurance premiums of health insurance policies depending on health degrees of pets is not handled sufficiently. Other than pets that are raised by individuals, this problem also applies to animals other than human, as exemplified by animals that are raised in zoos.

### [Configuration Example of Specimen Examination Management System or Like for Animals Other Than Human]

The specimen examination management system 10, specimen examination management system 10A and specimen examination management system 10B described using Figure 1 to Figure 19 can be applied to animals other than human by replacing the doctor with veterinarian, replacing the hospital with animal hospital, and replacing the examinee with pet owner. Here, the veterinarian corresponds to an example of the doctor.

The animal other than human may include animals that are raised in zoos, animals that are raised in ranches by livestock raisers, as exemplified by a chicken, a pig, a cow, and a horse, a race horse, and the like. The pet owner may include persons that manage the health of animals, as exemplified by a caretaker and a livestock manager.

As examples of the animal other than human, there are mammals such as a dog, a cat, a pig, a horse, a cow, a hamster, a guinea pig, a rabbit, a squirrel, and a ferret, birds, reptiles such as a snake and a lizard. As examples of the specimen of the animal other than human, there are blood, urine, stool, saliva, tunica mucosa, hair, and the like. As examples of the specimen examination item when the specimen is urine, there are urine pH, urine protein, uric blood, urine specific gravity, urine sugar, acesmin, aldehyde, ketone body, creatinine, and the like.

The specimen examination management system has been described in the embodiment, but a specimen examination management device, a specimen examination management method, and a program can be configured similarly to the specimen examination management system for human described above.

### [Function Effect of Specimen Examination Management System for Animals Other Than Human]

According to the specimen examination management system configured as described above, it is possible to obtain the same function effect as that for human.

### [Application Example of Health Degree Derivation]

In the above embodiments, a mode in which the health degree is derived based on the specimen examination information that is the specimen examination result has been exemplified. However, other than the specimen examination information, the derivation of the health degree may be based on the specimen examination subject information related to the specimen examination subject enumerated below.

For example, the health degree can be derived based on body measurement information that is expressed as a numerical value, as exemplified by the age, sex, height, weight, abdominal circumference, BMI, body fat percentage, visceral fat percentage, body temperature, heart rate, blood pressure, muscle mass and bone density of the examination subj ect.

As other examples of the body information that is expressed as a numerical value, there are daily consumed calories of the examination subject, nutrient intake rate, activity amount measured using an activity amount meter, daily salt intake, body balance, frailty determination, and the like. The health degree may be derived based on the numerical value itself, or the health degree may be derived based on temporal change in numerical value.

The health degree may be derived based on body information that is not expressed as a numerical value. As examples of the body information that is not expressed as a numerical value, there are inquiry relevant to body status, question relevant to body status, and the like.

Further, the health degree may be derived based on taste information relevant to the taste of the examination subject that includes whether the examination subject is a drinker, whether the examination subject is a smoker, and the like. The health degree may be derived based on a plurality of pieces of information in which the body information that is expressed as a numerical value, the body information that is not expressed as a numerical value, and the taste information are combined when appropriate.

That is, the examination management device 12 shown in Figure 1 includes a specimen examination subject information acquisition unit. The examination analysis unit 34 derives the health degree by analyzing at least one of the specimen examination subject information acquired using the specimen examination subject information acquisition unit and the specimen examination result.

### [Other Examples of Examination Result Applied in Derivation of Health Degree]

In the case where the specimen includes a so-called gene and exosome such as DNA (deoxyribonucleic acid), RNA (ribonucleic acid) and microRNA, for example, in the case where the specimen is saliva, blood, cells, and urine, the health degree can be derived using an SNPs analysis, a disease-associated gene examination, a liquid biopsy examination, an exosome examination, and the like.

Further, in the case where the specimen is stool, saliva, tunica mucosa or the like, the health degree can be derived using a result of an examination of a bacterial flora such as an intestinal bacterial flora.

In the analysis of the examination result, one examination item may be applied, or two or more examination items may be combined and applied. Furthermore, the above information that is applied in the derivation of the health degree may be combined with the examination result, and the analysis may be performed.

### [Application Example of Personal Authentication Information Acquisition]

In the personal authentication information acquisition, the following modes can be applied in addition to the mode of acquiring the pickup data obtained by picking up the person in question or the like using the pickup device. For example, it is possible to apply a mode of acquiring a picture of the specimen taking using a television telephone, a web meeting system or the like.

Further, it is possible to apply a mode in which a staff of the insurance company or the like photographs the specimen taking and acquires a moving image or a still image, and a mode of acquiring a record of identity confirmation execution at a drug store front, an insurance shop front or the like. Furthermore, it is possible to apply a mode of preserving a fingerprint stamped on an exclusive mount after the specimen taking and performing fingerprinting at the time of occurrence of a payment ground for insurance money, benefit money or the like. Instead of the fingerprinting based on the fingerprint preservation or in addition to this, it is possible to apply a DNA test based on preservation of body fluid such as blood.

As the personal authentication, it is possible to apply a knowledge authentication using an ID (identification), a password or the like that is confirmed in advance. As the personal authentication, it is possible to apply a possession authentication using an official document such as a driver license and a passport. In the possession authentication, it is possible to apply an IC card, a magnetic card, and optical data. As the personal authentication, it is possible to apply a biometric authentication using a fingerprint, a vein pattern, a face or the like that is confirmed in advance. The personal authentication may be performed while being combined with the above knowledge authentication and the like when appropriate.

### [Applying Example of Program Causing Computer to Function as Specimen Examination Management Device]

The above-described specimen examination management device and method can be realized by executing a program that realizes functions corresponding to the respective units in the specimen examination management device and functions corresponding to the respective steps in the specimen examination management method using a computer. For example, it is possible to configure a specimen examination management program that causes the computer to realize a specimen result acquisition function, a health degree computation function, an insurance information acquisition function, an insurance proposition creation function, and an insurance proposition output function.

It is possible to store the program that causes the computer to realize the above-described specimen examination management function, in a computer-readable information storage medium that is a non-transitory information storage medium, which is a tangible entity, and to provide the program through the information storage medium.

Further, instead of the mode of storing the program in the non-transitory information storage medium and providing the program, it is allowable to adopt a mode of providing a program signal through a network.

### [Combination of Embodiments, Modification and Like]

The constituent elements described in the above embodiments and the constituent elements described in the applying examples and the like can be combined and used when appropriate, and some constituent elements can be replaced.

In the above-described embodiments of the present invention, alteration, addition, and removal of components can be made when appropriate, without departing from the spirit of the present invention. The present invention is not limited to the above-described embodiments, and many modifications can be made within the technical idea of the present invention, by one ordinarily skilled in the art.

### {Reference Signs List}

10 specimen examination management system
10A specimen examination management system
10B specimen examination management system
11 examination insurance management integration device
12 examination management device
12B examination management unit
14 insurance management device
14B insurance management unit
16 examination organization terminal device
18 examinee terminal device
19 insurance subscriber terminal device
20 insurance management terminal device
22 network
30 examination information input unit
30A examination result input unit
30B insurance information input unit
30C examination acceptance information input unit
32 examination information output unit
32A examination result output unit
32B insurance information output unit
32C delivery information output unit
32D health degree information output unit
34 examination analysis unit
36 examination plan unit
38 examination database
40 control unit
42 memory
44 storage device
46 network controller
48 power supply device
49 bus
50 insurance management information input unit
52 insurance management information output unit
54 contract unit
56 insurance premium calculation unit
58 insurance database
102 demander information
120 examination result screen
122 examination date information
124 examination kit information
126 examination result list
140 insurance proposition screen
142 health degree information
146 insurance application selection button
160 application completion screen
162 application completion information
164 OK button
200 insurance proposition screen
206 re-examination hope selection button
220 re-examination acceptance completion screen
222 re-examination application completion information
224 OK button
240 insurance proposition screen
246 insurance condition review selection button
248 condition selection button
260 insurance condition review acceptance completion screen
262 application completion information
264 OK button
500 blood examination kit
502 sealing cap
504 cylinder
506 aspirator
508 lancet
510 bottle
512 absorber
S10 to S244 the respective steps of specimen examination management method

## Claims

1. A specimen examination management device comprising:
an examination result acquisition unit configured to acquire a result of a specimen examination using a specimen examination kit for each specimen examination subject;
a health degree computation unit configured to derive a health degree of the specimen examination subject based on the result of the specimen examination of the specimen examination subject that is acquired from the examination result acquisition unit, the health degree indicating a health state related to insurance subscription;
an insurance information acquisition unit configured to acquire insurance information including an insurance premium based on the health degree of the specimen examination subject, from an insurance premium calculation device configured to calculate the insurance premium based on the health degree;
an insurance proposition creation unit configured to create an insurance proposition including the insurance information that is acquired using the insurance information acquisition unit; and
an insurance proposition output unit configured to output the insurance proposition that is created using the insurance proposition creation unit.

2. The specimen examination management device according to claim 1, wherein
the health degree computation unit is configured to compute, as the health degree, an age difference resulting from subtracting a real age of the specimen examination subject from a health state age resulting from converting the health state of the specimen examination subject into an age.

3. The specimen examination management device according to claim 2, wherein:
the examination result acquisition unit is configured to acquire a result of a blood examination that adopts, as an examination item, at least one of glycohemoglobin, estimated glomerular filtration rate, albumin, aspartate aminotransferase, low-density lipoprotein cholesterol, alkaline phosphatase and total cholesterol; and
the health degree computation unit is configured to derive the health state age, from at least the examination item.

4. The specimen examination management device according to any one of claims 1 to 3, wherein
the examination result acquisition unit is configured to acquire the result of the specimen examination of the specimen examination subject, from an examination result storage device configured to store the result of the specimen examination using the specimen examination kit for each specimen examination subject.

5. The specimen examination management device according to claim 4, comprising the examination result storage device.

6. The specimen examination management device according to claim 5, comprising a personal authentication information acquisition unit configured to acquire personal authentication information about the specimen examination subject, wherein
the examination result storage device is configured to store the personal authentication information about the specimen examination subject that is acquired using the personal authentication information acquisition unit, in association with the result of the specimen examination.

7. The specimen examination management device according to claim 5 or 6, comprising a medical measurement value acquisition unit configured to acquire a medical measurement value of the specimen examination subject that is measured using a medical measurement apparatus, wherein
the examination result storage device is configured to store the medical measurement value of the specimen examination subject that is acquired using the medical measurement value acquisition unit, in association with the result of the specimen examination.

8. The specimen examination management device according to any one of claims 1 to 7, comprising a health degree information output unit configured to output health degree information including the health degree, to the insurance premium calculation device.

9. The specimen examination management device according to any one of claims 1 to 8, wherein
the insurance proposition output unit is configured to output the insurance proposition to a subject terminal device that is used by the specimen examination subject.

10. The specimen examination management device according to claim 9, comprising an examination result output unit configured to output the examination result that is acquired using the examination result acquisition unit, to the subject terminal device.

11. The specimen examination management device according to claim 9 or 10, comprising:
an intention confirmation information output unit configured to output intention confirmation information to the subject terminal device during an insurance contract continuation procedure allowing period, the intention confirmation information being information for confirming continuation intention for an insurance contract, the insurance contract continuation procedure allowing period being a period in which a continuation procedure of the insurance contract is allowed;
an intention information acquisition unit configured to acquire intention information indicating the continuation intention for the insurance contract of the specimen examination subject, the intention information being output from the subject terminal device; and
a delivery information output unit configured to output delivery information to a providing organization of the specimen examination kit, when the intention information acquisition unit acquires the intention information, the delivery information including a delivery address of the specimen examination kit for the specimen examination subject that outputs the intention information.

12. The specimen examination management device according to claim 11, wherein
the delivery information output unit is configured to output the delivery information to the providing organization of the specimen examination kit, when an insurance premium of an insurance after the insurance contract is continued is equal to or more than an insurance premium of an insurance under the contract, the delivery information including the delivery address of the specimen examination kit for the specimen examination subject that hopes for continuation of the insurance contract.

13. The specimen examination management device according to claim 11 or 12, wherein
the insurance information acquisition unit is configured to acquire information about an insurance premium of an insurance having a different contract condition including shortening of an insurance period, when an insurance premium of an insurance after the insurance contract is continued is equal to or more than an insurance premium of an insurance under the contract.

14. A specimen examination management system comprising a server device configured to be capable of being connected with a network,
the server device comprising:
an examination result acquisition unit configured to acquire a result of a specimen examination using a specimen examination kit for each specimen examination subject;
a health degree computation unit configured to derive a health degree of the specimen examination subject based on the result of the specimen examination of the specimen examination subject that is acquired from the examination result acquisition unit, the health degree indicating a health state related to insurance subscription;
an insurance information acquisition unit configured to acquire information about an insurance premium based on the health degree of the specimen examination subject, from an insurance premium calculation device configured to calculate the insurance premium based on the health degree;
an insurance proposition creation unit configured to create an insurance proposition including the information about the insurance premium that is acquired using the insurance information acquisition unit; and
an insurance proposition output unit configured to output the insurance proposition that is created using the insurance proposition creation unit.

15. The specimen examination management system according to claim 14, comprising an examination result storage device configured to be capable of being connected with the network, wherein:
the examination result storage device is configured to store the result of the specimen examination using the specimen examination kit for each specimen examination subject; and
the examination result acquisition unit is configured to acquire the result of the specimen examination of the specimen examination subject, from the examination result storage device.

16. The specimen examination management system according to claim 14 or 15, comprising a subject terminal device configured to be capable of being connected with the network, the subject terminal device being used by the specimen examination subject, wherein:
the subject terminal device comprises a display unit configured to display information that is output from the server device; and
the display unit is configured to display the insurance proposition that is output from the server device.

17. The specimen examination management system according to claim 16, wherein
the display unit is configured to display intention confirmation information during an insurance contract continuation procedure allowing period, the intention confirmation information being information for confirming continuation intention for an insurance contract that is output from the server device, the insurance contract continuation procedure allowing period being a period in which a continuation procedure of the insurance contract is allowed.

18. The specimen examination management system according to claim 16 or 17, wherein
the display unit is configured to display selection information that is output from the server device, when an insurance premium of an insurance after the insurance contract is continued is equal to or more than an insurance premium of an insurance under the contract, the selection information being information for selecting whether to perform a re-examination.

19. The specimen examination management system according to any one of claims 16 to 18, wherein
the display unit is configured to display information that is output from the server device, when an insurance premium of an insurance after the insurance contract is continued is equal to or more than an insurance premium of an insurance under the contract, the information being information about an insurance premium of an insurance having a different contract condition including shortening of an insurance period.

20. A specimen examination management method comprising:
an examination result acquisition step of acquiring a result of a specimen examination using a specimen examination kit for each specimen examination subject;
a health degree computation step of deriving a health degree of the specimen examination subject based on the result of the specimen examination of the specimen examination subject that is acquired in the examination result acquisition step, the health degree indicating a health state related to insurance subscription;
an insurance information acquisition step of acquiring insurance information including an insurance premium based on the health degree of the specimen examination subject, from an insurance premium calculation device configured to calculate the insurance premium based on the health degree;
an insurance proposition creation step of creating an insurance proposition including information about the insurance premium that is acquired in the insurance information acquisition step; and
an insurance proposition output step of outputting the insurance proposition that is created in the insurance proposition creation step.

21. A program causing a computer to realize:
an examination result acquisition function to acquire a result of a specimen examination using a specimen examination kit for each specimen examination subject;
a health degree computation function to derive a health degree of the specimen examination subject based on the result of the specimen examination of the specimen examination subject that is acquired using the examination result acquisition function, the health degree indicating a health state related to insurance subscription;
an insurance information acquisition function to acquire insurance information including an insurance premium based on the health degree of the specimen examination subject, from an insurance premium calculation device configured to calculate the insurance premium based on the health degree;
an insurance proposition creation function to create an insurance proposition including information about the insurance premium that is acquired using the insurance information acquisition function; and
an insurance proposition output function to output the insurance proposition that is created using the insurance proposition creation function.

22. A specimen examination management device comprising:
a health degree computation unit configured to derive a health degree of a specimen examination subject based on at least one of a result of a specimen examination of the specimen examination subject and specimen examination subject information, the health degree indicating a health state related to insurance subscription, the specimen examination subject information being information related to the specimen examination subject;
an insurance information acquisition unit configured to acquire insurance information including an insurance premium based on the health degree of the specimen examination subject, from an insurance premium calculation device configured to calculate the insurance premium based on the health degree;
an insurance proposition creation unit configured to create an insurance proposition including the insurance information that is acquired using the insurance information acquisition unit; and
an insurance proposition output unit configured to output the insurance proposition that is created using the insurance proposition creation unit.

23. The specimen examination management device according to claim 22, wherein
the specimen examination subject information includes at least one of body information relevant to a body of the specimen examination subject and taste information relevant to a taste of the specimen examination subject.

24. A non-transitory computer-readable storage medium causing a computer to realize functions when a command stored in the storage medium is read by the computer, the functions comprising:
an examination result acquisition function to acquire a result of a specimen examination using a specimen examination kit for each specimen examination subject;
a health degree computation function to derive a health degree of the specimen examination subject based on the result of the specimen examination of the specimen examination subject that is acquired using the examination result acquisition function, the health degree indicating a health state related to insurance subscription;
an insurance information acquisition function to acquire insurance information including an insurance premium based on the health degree of the specimen examination subject, from an insurance premium calculation device configured to calculate the insurance premium based on the health degree;
an insurance proposition creation function to create an insurance proposition including information about the insurance premium that is acquired using the insurance information acquisition function; and
an insurance proposition output function to output the insurance proposition that is created using the insurance proposition creation function.
